# EUROPÄISCHE PATENTSCHRIFT

(11) **EP 1 194 111 B1**
(45) Veröffentlichungstag und Bekanntmachung des Hinweises auf die Patenterteilung: **06.09.2006**
(21) Anmeldenummer: 00951366.4
(22) Anmeldetag: 10.07.2000
(51) Int. Cl.: A61K 8/04, A61K 8/34, A61K 8/35, A61K 8/37, A61Q 1/06, A61Q 5/00, A61Q 5/02, A61Q 17/04, A61Q 19/00

(54) **MITTEL MIT UV-STRAHLUNG ABSORBIERENDER UND/ODER REFLEKTIERENDER WIRKUNG ZUM SCHUTZ VOR GESUNDHEITSSCHÄDLICHER UV-STRAHLUNG UND STÄRKUNG DER NATÜRLICHEN HAUTBARRIERE**
UV RADIATION REFLECTING OR ABSORBING AGENTS, PROTECTING AGAINST HARMFUL UV RADIATION AND REINFORCING THE NATURAL SKIN BARRIER
AGENTS A EFFET REFLECHISSANT OU ABSORBANT DU RAYONNEMENT UV, PROTEGEANT CONTRE LE RAYONNEMENT UV NOCIF ET RENFOR ANT LA BARRIERE CUTANEE NATURELLE

(30) Priorität: 13.07.1999 DE 19932156; 31.03.2000 DE 10016155
(43) Veröffentlichungstag der Anmeldung: 10.04.2002
(73) Patentinhaber: CLR Chemisches Laboratorium Dr. Kurt Richter GmbH, 12159 Berlin (DE)
(72) Erfinder: MÜLLER, Rainer, Helmut, D-12161 Berlin (DE); WISSING, Sylvia, D-10707 Berlin (DE); MÄDER, Karsten, D-13187 Berlin (DE)
(74) Vertreter: UEXKÜLL & STOLBERG
(86) Internationale Anmeldenummer: PCT/EP2000/006534
(87) Internationale Veröffentlichungsnummer: WO 2001/003652

(56) Entgegenhaltungen:
- EP-A- 0 379 409
- EP-A- 0 529 396
- EP-A- 0 573 229
- WO-A-00/67728
- WO-A-98/46200
- DE-A- 4 131 562
- US-A- 5 496 565
- US-A- 5 733 531
- US-A- 5 904 932

## Beschreibung

Die Erfindung betrifft die Verwendung feste, polymorphen Lipidpartikel zum Herstellung von Mittel mit UV-Strahlung absorbierender und/oder reflektierender Wirkung zur Anwendung auf der Haut, Schleimhäuten, Kopfhaut und Haaren zum Schutz vor gesundheitsschädlicher UV-Strahlung und Stärkung der natürlichen Hautbarriere.

Mit Zunahme des Ozon-Loches sowie weltweiter Abnahme der Dicke der Ozonschicht und daraus resultierender zunehmender Exposition der menschlichen Haut mit gesundheitsschädlicher UV-Strahlung steigt der Bedarf und die Notwendigkeit an Mitteln, die die Haut vor UV-Strahlung schützen, das heißt die UV-Strahlung abschwächen oder im Idealfall vollständig blockieren. Die gesundheitsschädliche Wirkung von UV-Strahlen macht sich unter anderem in Form von Hautkrebs (z.B. Melanom) bemerkbar. Die Zunahme der UV-Strahlenbelastung der Haut in den letzten Jahren hat zu einer starken Zunahme an Hautkrebserkrankungen geführt. Während die Inzidenz einiger Krebsarten rückläufig ist, ist infolge der zunehmenden UV-Belastung das Melanom der Haut eine der Krebsarten mit der höchsten Steigerungsraten. Alle 5 Jahre verdoppelt sich die Neuerkrankungsrate des malignen Melanoms (E. Wolf, Angst vor der Sonne, Pharmazeutische Zeitung 144, 1839-1843). Insbesondere ist die Bevölkerung von Ländern mit hoher und intensiver Sonnenbelastung betroffen, z.B. Ozonloch über Südchile, Neuseeland und Australien. So liegt die Inzidenz des malignen Melanoms in Australien fünfmal höher als in Mitteleuropa (E. Wolf, Angst vor der Sonne, Pharmazeutische Zeitung 144, 1839-1843).

Der traditionelle Lösungsansatz zum Schutz vor UV-Strahlung ist die Einarbeitung von UV-Strahlung absorbierenden Molekülen (sog. UV-Blocker) in Cremes oder Lotionen, die zum Sonnenschutz auf die Haut aufgetragen werden und dort über Stunden verbleiben (N.J. Lowe, Photoprotection, Seminars in Dermatology, Vol. 9, No. 1, 1990, 78-83). Genaugenommen ist die Bezeichnung UV-Blocker irreführend, da die UV-Strahlung nicht vollständig blockiert sondern nur, in Abhängigkeit der Konzentration und chemischen Natur der eingesetzten Substanzen, mehr oder weniger stark abgeschwächt wird.

Einer der Nachteile der molekularen UV-Blocker ist, daß sie analog zu in eine Creme eingearbeiteten Arzneistoffen in die Haut hineindiffundieren. Bei Arzneistoffen ist dies erwünscht bei UV-Blockern nicht, da sie unerwünschte Nebenwirkungen hervorrufen.

Nebenwirkungen von UV-Blockern sind z.B. Photosensibilisierung wie Photoallergie und Phototoxizität sowie Hautirritationen. Bei empfindlichen Personen wird dabei eine Fremdsubstanz - oft ein topischer chemischer UV-Filter - durch UV-Strahlung aktiviert, und diese aktivierte form ruft dann diese Reaktion hervor (E. Wolf, Angst vor der Sonne, Pharmazeutische Zeitung 144, 1839-1843). Hautirritationen sind bei einigen Substanzklassen (Salicyliden) so stark sind, daß sie nicht auf der Haut angewandt werden können. Daraus ergibt sich die Forderung, Penetration in die Haut zu minimieren (E. Mariani, C. Neuhoff, A. Bargagna, F. Bonina, M. Giacchi, G. De Guidi, A. Velardita, Synthesis, in vitro percutaneous absorption and phototoxicity of new benzylidene derivatives of 1,3,3-trimethyl-2-oxabicyclo(2.2.2)octan-6-6one as potential UV sunscreens, Int. J. Pharm. 161, 65-73). Bei guter Löslichkeit im Vehikel (z.B. molekulare UV-Blocker in der Ölphase einer Lotion oder Creme) kommt es jedoch sehr leicht zu einer Penetration in die Haut (U. Hagedorn-Leweke, B.C. Lippold, Accumulation of sunscreens and other compounds in keratinous substrates, Eur. J. Pharm. Biopharm. 46, 215-221). Die Hautpenetration molekularer UV-Blocker stellt somit ein ungelöstes Problem dar. Verstärkt kommt daher die Forderung, physikalisch wirkende Lichtschutzfilter einzusetzen, die nicht in die Haut penetrieren (E. Wolf, Angst vor der Sonne, Pharmazeutische Zeitung 144, 1839-1843).

Weitere Probleme sind die toxikologische Prüfung der UV-Blocker nach den Richtlinien für Kosmetika, die weniger streng sind als die Prüfung für Arzneimittel. UV-Blocker können sich unter UV-Strahlung zersetzen. So entstehen reaktive Zersetzungsprodukte, die insbesondere bei Hautpenetration toxikologisch bedenklich sein können. Für einige UV-Blocker ist bekannt, daß sie spezifisch an Keratinstrukturen der Haut binden und daher nur schwer abzuwaschen sind (U. Hagedorn-Leweke, B.C. Lippold, Accumulation of sunscreens and other compounds in keratinous substrates, Eur. J. Pharm. Biopharm. 46, 215-221). Ein ideales Sonnenschutzmittel sollte zur Minimierung der Toxizität nach dem Sonnenbad abwaschbar sein.

Die Penetration - und damit die Nebenwirkungen können besonders ausgeprägt sein, wenn die UV-Blocker in der wäßrigen Phase von Öl-in-Wasser (O/W) Cremes oder Lotionen gelöst sind. Die Phase in direktem Kontakt mit der Haut (Wasserphase) hat eine hohe Konzentration an UV-Blocker, so daß der Konzentrationsgradient Wasserphase-Haut groß ist, was nach dem ersten FICK schen Diffusionsgesetz die Penetration in die Haut fördert. Dies ist ein Effekt der bei transdermalen therapeutischen Pflastern in der Pharmazie gezielt ausgenutzt wird, bei UV-Blockern jedoch unerwünscht ist und minimiert werden muß.

Ein Ansatz zur Minimierung der Hautpenetration ist die Verwendung von lipophilen UV-Blockern mit geringer Wasserlöslichkeit. Diese sind in der Ölphase der Creme oder Lotion gelöst. Die Wasserphase enthält eine deutlich geringere Konzentration an UV-Blocker. Dies kann bei günstiger chemischer Struktur des UV-Blockers die Penetration in die Haut aufgrund des nun geringeren Konzentrationsgradienten verlangsamen, vermeidet sie aber nicht. Aus der Wasserphase in die Haut diffundierter UV-Blocker wird durch Diffusion von weiterem UV-Blocker aus der Ölphase in die Wasserphase ersetzt. Die Umverteilung in die Wasserphase erfolgt nach dem NERNST' schen Verteilungskoeffizienten einer Substanz.

Um die Nebenwirkung von molekularen UV-Blockern zu umgehen verfolgte man den Ansatz partikuläre UV-Blocker einzusetzen. Ein Beispiel ist das breit eingesetzte anorganische Titandioxid (B.L. Diffey, P.M. Farr, Sunscreen protection against UVB, UVA and blue light; an in vivo and in vitro comparison, British Journal of Dermatology 124, 1991, 258-263). Die Grundidee war, daß die Partikel aufgrund ihrer Größe nicht in die Haut diffundieren und somit keine Nebenwirkungen hervorrufen sollten. Nach dem Sonnenbad sollten die Partikel mit der normalen Körperreinigung von der Haut abgewaschen werden (z.B. Dusche).

Partikuläre UV-Blocker wie Mikropigmente (z.B. Titandioxid) haben einen sofort augenfälligen kosmetischen Nachteil in Präparaten mit hohem Lichtschutzfaktor. Bei der notwendigen hohen Menge an Pigment tritt ein Weißeffekt auf (E. Wolf, Angst vor der Sonne, Pharmazeutische Zeitung 144, 1839-1843). Besonders effektiv erwiesen sich sehr kleine Titandioxidpartikel (B.L. Diffey, P.M. Farr, Sunscreen protection against UVB, UVA and blue light; an in vivo and in vitro comparison, British Journal of Dermatology 124, 1991, 258-263), so daß sie entsprechend Einsatz mit bis zu 25 % in Kosmetika fanden. Allerdings hat man auch bei Titandioxidpartikeln Wechselwirkungen und Nebenwirkungen mit der Haut gefunden (R.G. van der Molen et al, Efficacy of micronized titanium dioxide-containing compounds in protection against UVBinduced immunosuppression in humans in vivo, Journal of Photochemistry and Photobiology 44, 2, 1998, 143-150), und es kann nicht mehr ausgeschlossen werden, daß Titandioxid in die Haut eindringt (R.G. van der Molen, Tape stripping of human stratum corneum yields cell layers that originate from various depths because of furrows in the skin, Archives of dermatological research, 289, 9, 1997, 514-518). So zeigte sich beispielsweise, daß Titandioxid die Bildung freier Radikale photokatalysieren kann (W.G. Wamer, Oxidative damage to nucleic acids photosensitized by titanium dioxide, Free Radical Biology and Medicine, 23,6, 1997, 851-858), was sowohl in der Haut als auch auf der Haut bzw. während der Lagerung kritisch zu betrachten ist.

Die US-A-5 733 531 beschreibt eine Sonnenblockerkomponente, die eine Vielzahl von Teilchen mit einem Durchmesser im Bereich von etwa 0,01 bis 100 µm aus einer Matrix, die Wachs enthält, und einer UV-Schutzverbindung in dem Wachs umfaßt, wobei die Teilchen lösungsmittelfrei und in einem dermatologisch akzeptablen flüssigen Träger dispergierbar sind. Das Wachs kann ein Material sein, das aus natürlichen oder synthetischen Wachsen ausgewählt ist.

Die US-A-5 496 565 beschreibt Mikrosphären einer Zusammensetzung, die im wesentlichen aus 1 bis 20% eines Fettalkohols, 1 bis 20% Cetylpalmitat und 1 bis 20% hochdispersem Siliciumdioxid besteht. Die Mikrosphären bzw. Mikropartikel weisen dabei eine Größe von 1 bis 1000 µm auf. Zur Herstellung eines UV-Schutzmittels sind zusätzlich noch UV-Schutzmaterialien erforderlich. Darüber hinaus ist die Anwesenheit von hochdispersen Siliciumdioxid zwingend erforderlich.

Die WO 98 46200 A1 beschreibt ein Verfahren zur Herstellung eines Abgabesystems für mindestens eine feste oder flüssige organische Sonnenschutzsubstanz, bei dem mindestens ein festes organisches Wachsschmiermittel, das mit mindestens einem Sonnenschutzmittel bei einer Temperatur im Bereich von 70 bis 150°C mischbar ist ausgewählt wird, erhitzt wird, gerührt wird und abgekühlt wird, um eine feste Mischung zu bilden. Anschließend wird gemahlen, um eine Pulvermischung herzustellen. Der Größenbereich des gebildeten Pulvers liegt im Mikrometerbereich.

Aus der US-A-5 904 932 sind Nanoteilchen bekannt, die mindestens ein festes Paraffinlipid umfassen. Als Paraffinlipide kommen eine Reihe von Verbindungen in Frage, wobei Harzparaffin als typischer Vertreter der geeigneten Materialien bevorzugt wird. Die genannten Teilchen werden zur Herstellung von topisch anwendbaren Arzneimitteln verwendet.

Ferner sind aus der DE 41 31 562 A1 Arzneistoffträger aus festen Lipid- oder Lipoidteilchen bekannt, die einen Durchmesser von 10 bis 1000 nm aufweisen, sogenannte SLN. Diese werden dort zur Applikation von Arzneimittelwirkstoffen verwendet.

Zusammenfassend kann somit festgestellt werden, daß angesichts der intensiveren Strahlenbelastung bei gleichzeitiger vermehrter Anwendung ein Bedarf sowohl an effizienteren als auch toxikologisch besser verträglichen Sonnenschutzmitteln besteht, insbesondere auch für hochempfindliche Hautareale.

Aufgabe der Erfindung ist es, ein besser verträgliches Mittel zum Schutz gegen schädliche UV-Strahlung bereitzustellen, daß die oben beschriebenen Nachteile vermeidet und insbesondere die Umverteilung von molekularen UV-Blockern aus der dispergierten Phase (z.B. Öltropfen einer Lotion) in die äußere (disperse) Phase stark minimiert bzw. vermeidet.

Die Aufgabe wird erfindungsgemäß durch die Verwendung von festen, polymorphen, kristallinen oder teilkristallinen Lipidpartikeln oder einer Zusammensetzung, die feste, polymorphe kristalline oder teilkristalline Lipidpartikel, gegebenenfalls als feste innere Phase (Lipidphase) dispergiert in einer äußeren flüssigen Phase umfaßt, als Mittel zum Schutz vor UV-Strahlung, wobei die Lipidpartikel eine Größe, ausgedrückt als Mittelwert der Hauptpopulation, im Bereich von 10 nm bis 1000 nm aufweisen und während der Aufheizphase in der Wärmekalorimetrie (DSC - Differential Scanning Calorimetry) einen endothermen Peak oberhalb 20 °C zeigen, zur Herstellung von Mitteln mit UV-Strahlung absorbierender und/oder reflektierender Wirkung zur Anwendung auf der Haut, Schleimhäuten, Haaren und Kopfhaut zum Schutz vor gesundheitsschädlicher UV-Strahlung und Stärkung der natürlichen Hautbarriere gelöst.

Je nach Erfordernis werden in die festen Lipid- partikel UV-Blocker eingearbeitet. So hergestellte Sonnenschutzmittel sind keine Emulsionen mehr, sondern stellen technologisch eine Suspension dar.

Der Ausdruck "polymorph" bezieht sich dabei auf die Eigenschaften von Molekülen in unterschiedlichen Modifikationen vorliegen zu können. Die polymorphen Formen können kristallin (vollkristallin) (z.B. β-, βi-Modifikation) oder flüssig-kristallin (z.B. α-Modifikation) sein. Bei Vorliegen mehrerer verschiedener Modifikationen (kristallin und flüssig-kristallin) kann sich daher auch eine teilkristalline Form der erfindungsgemäßen Teilchen ergeben. Liegen nur Modifikationen mit kristalliner Struktur vor, sind die Teilchen ebenfalls kristallin. Liegen in den erfindungsgemäßen Teilchen sowohl Bereiche mit Modifikationen mit kristalliner Struktur als auch Bereiche mit mit flüssig-kristalliner Struktur vor, sind die Teilchen insgesamt teilkristallin.

Bei den angegebenen Partikelgrößen handelt es sich um den Mittelwert der Hauptpopulation. Bei kleinen Partikeln sind es die mittleren Durchmesser gemessen mit Photoenkorrealtionsspektroskopie (PCS, Meßbereich 3 nm bis 3 µm) oder Laserdiffraktometrie (LD).

Im folgenden wird die Erfindung der Einfachheit halber anhand von Ausführungsformen beschrieben, die Lipide enthalten. Erfindungsgemäß sind jedoch auch Ausführungsformen umfaßt, die Lipide und Polymer enthalten. Die Auführungen gelten daher auch für diese alternativen Ausführungsformen.

Bei der Bestimmung der UV-blockierenden Wirkung wurde nun überraschenderweise festgestellt, daß im Vergleich zu Emulsionen bereits die Lipidpartikel selbst ohne eingearbeiteten molekularen UV-Blocker eine UV-Strahlung blockierendende Wirkung haben (Beispiele 1-3). Somit eröffnet sich sogar die Möglichkeit auf die Verwendung toxikologisch ungünstiger molekularer UV-Blocker zu verzichten.

Die UV-blockierende Wirkung der festen Lipidpartikel nimmt mit steigender Konzentration zu, so daß der gewünschte Lichtschutzfaktor über die Partikelkonzentration eingestellt werden kann (Beispiel 4).

Die UV-blockierende Wirkung ist auch eine Funktion der Partikelgröße. Lipidnanopartikel waren bei identischer Lipidkonzentration in der Suspension effektiver als 4,6 µm große Mikropartikel (Beispiel 5). Dies konnte durch Untersuchungen an Polymerpartikeln unterschiedlicher Größe bestätigt werden. Partikel im Bereich von ca. 500 nm bis 1000 nm zeigten den stärksten UV-blockierenden Effekt, sehr kleine Nanopartikel (60 nm) und größere Mikropartikel waren weniger effektiv (Beispiel 7).

Es können auch Mischungen von Lipiden und Polymeren verwendet werden. Geeignet als Polymer sind im allgemeinen bei Raumtemperatur (20°C) feste Polymere wie Polystyrole, Polyacrylate, Polymethacrylate, Polycarbonate, Polyamide, Polyurethane, Polyhydroxybuttersäure (PHB), Polyhydroxyvaleriansäure (PHV), Cellulosen und Cellulosederivate, insbesondere Cellulosehydrate, Polylactide (PLA), Polyglycolide (PPGA) und deren Copolymere (PLA/GA) einzeln oder in Mischung.

Lipidmikropartikel sind jedoch ökologisch am vorteilhaftesten, speziell wenn sie aus nachwachsenden Rohstoffen (z.B. pflanzlichen Lipiden) hergestellt werden, gleichzeitig sind sie wirtschaftlich am kostengünstigsten.

Die Lipidpartikel-Suspension kann direkt auf die Haut aufgetragen werden, falls gewünscht kann zur Viskositätserhöhung ein Gelbildner zugesetzt werden. Alternativ können die Partikel auch in Lotionen und Cremes eingearbeitet werden. Sie sind darin physikalisch stabil und lösen sich nicht in der Ölphase auf (Beispiel 6).

Nach Austreichen auf ein Oberfläche bilden die Lipidpartikel gleichmäßige Filme, eine Voraussetzung für eine effektive UV-blockierende Wirkung (Beispiel 8). Es kam dabei nicht wie befürchtet zur Ausbildung löcheriger, poröser Filme, sondern zur Bildung eines geschlossenen Films (Beispiel 18). Dieser Lipidfilm stärkt die natürliche Hautbarriere, insbesondere wenn bereits ein geschädigter natürlicher Lipidfilm auf dem Stratum Corneum vorliegt.

In die Lipidpartikel können auch UV-Blocker eingearbeitet werden, um die UV-blockierende Wirkung zusätzlich zu verstärken (Beispiele 11 und 12). Dabei wurde überraschenderweise gefunden, daß die Wirkung von Lipidpartikeln und UV-Blocker nicht nur additiv, sondern auch synergistisch sein kann (Beispiel 17).

Da Sonnenexposition ein Streß für die :Haut bedeutet kann es sinnvoll sein, in die Lipidpartikel hautpflegende Substanzen wie Retinolpalmitat oder Antioxidantien wie Tocopherol einzuarbeiten. Es können auch beide Wirkstoffgruppen gleichzeitig verarbeitet werden.

Die erfindungsgemäß verwendeten Lipidapartikel können auch eingesetzt werden, um die Wechselwirkung von anorganischen oder organischen Pigmenten mit der Haut zu minimieren. Analog zu den molekularen UV-Blocker werden die Pigmente (pigmentäre oder partikuläre UV-Blocker) in die feste Lipidmatrix eingeschlossen. Die Einarbeitung kann auch problemlos in Lipidpartikel im unteren Nanometerbereich erfolgen (z.B. 200 nm Partikel), da viele Pigmente sehr klein sind (ca. 10-40 nm bei Magnesiumschichtsilikaten wie Aerosil, ca. 15-20 nm bei Titandioxid) (Beispiele 15 und 16).

Möglich ist auch die Einarbeitung einer Kombination von molekularen UV-Blockern und partikulären UV-Blockern (Pigmente) sowie der gleichzeitige Zusatz von hautpflegenden Wirkstoffen sowie Antioxidantien, entweder in die feste Lipidmatrix oder zur äußeren Phase der Lipidpartikeldispersion.

Die erfindungsgemäß verwendeten Lipidpartikeldispersionen können auch Emulgator-frei hergestellt werden, was wichtig ist zur Vermeidung der Mallorca-Akne. Die Mallorca-Akne wird nicht allein durch UV-A Strahlung ausgelöst sondern durch die ihre Wechselwirkung mit Emulgatoren in Kosmetika (E. Wolf, Angst vor der Sonne, Pharmazeutische Zeitung 144, 1839-1843).

Es ergeben sich zusätzlich Anwendungsmöglichkeiten auf Kopfhaut und Haar (z.B. zur Vermeidung von Sonnenbrand bei lichtem Haar, Vermeidung von Ausbleicheffekten bei Haaren). Speziell zur Erhöhung der Adhäsion an die negativ geladenen Haare können die Lipidpartikel durch Verwendung entsprechender Tenside positiv geladen hergestellt werden.

Zur Erhöhung der Akzeptanz des Mittels zur UV-Absorption können in die Lipidpartikel natürliche, synthetische oder halbsynthetische Duftstoffe eingearbeitet werden, z.B. Parfüms, ätherische Öle oder Pheromone.

Beispiele für Parfüms sind Allure, Coco, Egoiste, Chanel No. 5, 19, 22 von Chanel, Miss Dior, Dune, Diorissime oder Fahrenheit von Dior, Roma, Laura, Venezia von Laura Biagotti, L' air du temps von Nina Ricci, Chalimar von Guerlain, Tresor von Lancome, Gio von Armani, Escape, Obsession, CK One, CK be, Eternity von Calvin Klein, Berlin, Joop, Rococo, All about Eve, What about Adam, Nightflight von Joop, KL, Lagerfeld, Jako von Karl Lagerfeld, Extreme von Bulgari.

Beispiele für ätherische Öle sind Zitronenöl, Rosenöl, Lavendelöl, Bergamottöl, Melissenöl, Nelkenöl, Zimtöl, Orangenöl, Jasminöl, Rosmarinöl, Anisöl, Pfefferminzöl, Sandelholzöl Ylang-Ylang-Öl oder deren isolierte Inhaltsstoffe wie z.B. 1,8-Cineol, Menthol, Terpinhydrat, Limonen, α-Pinen, Eugenol.

Beipiele für Pheromone sind insbesondere menschliche Pheromone wie Androstenon oder Androstenol.

Die Duftstoffe können in die Lipidpartikel alleine oder in Kombination mit beispielsweise UV-Blockern wie z.B. partikulären oder molekularen UV-Blocker eingearbeitet werden.

Zur Anwendung des Mittels zur UV-Absorption in Insekten-belasteten Gebieten (z.B. Moskitos an indischen Stränden) können in die Lipidpartikel Repellents eingearbeitet werden. Beispiele für Repellents sind natürliche Repellents wie Citrusöle, Eukalyptusöl und Campher oder synthetische Repellents wie N,N-Diethyltoluamid (DEET), Dibutylphthalat, Dimethylphthalat, 2-Ethyl-1,3-hexandiol. Die Repellents können in die Lipidpartikel alleine oder in Kombination mit Duftstoffen und/oder UV-Blockern wie z.B. partikulären oder molekularen UV-Blockern eingearbeitet werden.

Die Erfindung wird im Folgenden ausführlicher anhand der angefügten Figuren 1 bis 18 und von Beispielen beschrieben.

Bei den Fig. 1-5 und 7-17 sind in der Abszisse jeweils die Wellenlänge [nm] und in der Ordinate jeweils die Absorptionen aufgetragen.
- Abbildung 1:: Spektrophotometrische Scans von wäßrigen Dispersionen (Beispiel 1).
Cetylpalmitat - TegoCare, (2) Miglyol - Tego Care, (3) Cetylpalmitat, (4) TegoCare
- Abbildung 2:: Spektrophotometrische Scans von wäßrigen Dispersionen (Beispiel 2).
Stearylalkohol - Tween 80, (2) Miglyol - Tween 80, (3) Stearylalkohol, (4) Tween 80
- Abbildung 3:: Spektrophotometrische Scans von Lipidfilmen (Beispiel 3)
Cetylpalmitat - Tego Care, (2) Miglyol - Tegocare
- Abbildung 4:: Spektrophotometrische Scans von Lipidfilmen (Beispiel 4)
10 % Cetylpalmitat, (2) 20 % Cetylpalmitat, (3) 30 % Cetylpalmitat, (4) 40 % Cetylpalmitat
- Abbildung 5:: Spektrophotometrische Scans von Lipidfilmen (Beispiel 5)
Nanopartikel (d50 % 138 nm), (2) Mikropartikel (d50 % 4,6 µm)
- Abbildung 6:: Thermogramm einer Cetylpalmitat SLN-Dispersion (oben) im vergleich mit der Dispersion eingearbeitet in eine O/W-Creme (mitte) (normiert auf den Anteil an SLN-Dispersion sowie die reine O/W-Creme ohne SLN (unten)
- Abbildung 7:: Spektrophotometrische Scans von Lipidfilmen zur Bestimmung der Filmgleichmäßigkeit (Beispiel 7) (1) ― (6) : unterschiedliche Stellungen der Küvette im Halter
- Abbildung 8:: Spektrophotometrische Scans von Lipidfilmen (Beispiel 8)
Cetylpalmitat - TegoCare ― Eusolex 4360, (2)
Cetylpalmitat - TegoCare
- Abbildung 9:: Spektrophotometrische Scans von Lipidfilmen (Beispiel 9)
10 % Eusolex 4360, (2) 5 % Eusolex 4360, (3) 1 %
Eusolex 4360
- Abbildung 10:: Spektrophotometrische Scans von Lipidfilmen (Beispiel 10)
Cetylpalmitat ― TegoCare ― Eusolex 4360, (2)
Miglyol - TegoCare ― Eusolex 4360
- Abbildung 11:: Spektrophotometrische Scans von Lipidfilmen (Beispiel 11)
Mikrometerpartikel mit Eusolex 4360 (d50 % 12 µm), (2) Mikrometerpartikel (d50% 4,6 µm), (3)
Nanometerpartikel mit Eusolex 4360 (d50 % 138 nm)

- Abbildung 12:: Spektrophotometrische Scans von Lipidfilmen (Beispiel 12)
Cetylpalmitat - TegoCare - Eusolex 4360 - Vitamin A palmitat
Cetylpalmitat - TegoCare - Eusolex 4360
- Abbildung 13:: Spektrophotometrische Scans von Lipidfilmen (Beispiel 13)
Cetylpalmitat - TegoCare - Eusolex 4360 - Vitamin E
Cetylpalmitat - TegoCare - Eusolex 4360
- Abbildung 14:: Spektrophotometrische Scans von Lipidfilmen (Beispiel 14)
Cetylpalmitat ― TegoCare, (2) Cetylpalmitat-TegoCare - Aerosil 200
- Abbildung 15:: Spektrophotometrische Scans von Lipidfilmen (Beispiel 15)
Cetylpalmitat - TegoCare ― Eusolex 4360
Cetylpalmitat - TegoCare ― Eusolex 4360 - Aerosil 200
- Abbildung 16:: Spektrophotometrische Scans von Lipidfilmen (Beispiel 16)
Cetylpalmitat - TegoCare (Eigenabsorption Lipidpartikel)
Errechnete Absorption von Cetylpalmitat - Tego-Care - Eusolex 4360 - Lipidpartikeln
Praktisch ermittelte Absorption von Cetylpalmitat - TegoCare - Eusolex 4360 - Lipidpartikeln (Synergismus)
- Abbildung 17:: Elektronenmikroskopie-Aufnahme des geschlossenen Lipidfilms von Beispiel 17

### Detaillierte Beschreibung der Erfindung

Erfindungsgemäß wurde gefunden, daß zum Schutz vor UV-Strahlung eine Suspension fester Lipidpartikel verwendet werden kann, wobei die Suspension in einer äußeren Phase (z.B. Wasser) dispergierte Lipidpartikel umfaßt, die bis 20 °C eine feste Matrix besitzen. Die Lipidpartikel sind dadurch gekennzeichnet, daß im Gegensatz zu einer Öl-in-Wasser Emulsion beim Aufheizen in der Differential Scanning Calorometry (DSC) oberhalb 20° C ein Schmelzpeak erhalten wird. Die Lipidpartikel können (voll)kristallin aber auch teilkristallin sein (z.B. bei Vorhandensein eines Anteils an α-Modifikation im Lipid). Die Partikelgröße beträgt 10 bis 1000nm.

Die UV-blockierende Wirkung des Mittels wurde durch Messung der UV-Absorption der Lipidpartikeldispersion in einem UV-Spektrophotometer vermessen. Kriterium war die verringerte Durchlässigkeit des Lipidpartikeldispersion für UV-Strahlung im Wellenlängenbereich bis zu 280 nm (UV C), von 280 nm bis 315 nm (= UV B) und von 315 nm bis 400 nm (UV A). Als weiterer Test zur Quantifizierung der UV-blockierenden Wirkung war die Bestimmung der verringerten Durchlässigkeit von Partikelfilmen unter Benutzung des Standardtestes mit Transpore Tape (B.L. Diffey, P.M. Farr, Sunscreen protection against UVB, UVA and blue light; an in vivo and in vitro comparison, British Journal of Dermatology 124, 1991, 258-263). Die Partikelfilme wurden durch Ausstreichen der Partikeldispersion auf Transpore Tape und anschließende Lufttrocknung hergestellt. Die so hergestellten Filme wurden dann auf eine Seite einer Quarz-Küvette aufgeklebt und die UV-Durchlässigkeit im Photometer bestimmt. Die Messungen erfolgten gegen die entsprechenden Referenzen, z.B. O/W Emulsion gleichen Lipidgehaltes sowie UV-Blöcker eingearbeitet in die Ölphase einer Emulsion.

Die Herstellung der Lipidpartikel erfolgt durch Dispergierung oder Präzipitation des Lipids, wobei in Lehrbüchern der Pharmazie und Verfahrenstechnik beschriebene allgemein bekannte Methoden eingesetzt werden. Bei der Dispergierung zerteilt man grobdisperse Lipide durch mechanische Verfahren. Die Lipide können sich hierbei im festen Aggregatzustand (z. B. Mörsermühle) oder im flüssigen Aggregatzustand befinden (z.B. Emulgierung geschmolzener Lipide durch Rührer). Zur Herstellung der Lipidpartikeldispersion können die Lipide zuerst zerkleinert und anschließend in der äußeren (z.B. wäßrigen) Phase dispergiert werden oder alternativ direkt in der äußeren Phase zerkleinert werden. Bei Zerkleinerung des Lipides vor Dispergierung in der äußeren Phase können z.B. eingesetzt werden: Gasstrahlmühle, Rotor-Stator-Kolloidmühle und Mörsermühle.

Die Dispergierung des Lipids in der äußeren Phase kann entweder im festen Zustand (Kaltdispergierung) oder im flüssigen Zustand (Heißdispergierung) erfolgen. Bei der Kaltdispergierung wird das gepulverte Lipid in einer wäßrigen Tensidlösung dispergiert (Rohdispersion) und dann mit einem entsprechenden Gerät weiterverarbeitet. Bei der Heißdispergierung wird das Lipid geschmolzen und in die auf gleicher Temperatur erhitzte äußere Phase gegossen und darin dispergiert (Rohemulsion). Die erhaltene Rohemulsion wird dann mit einem weiteren Dispergiergerät bearbeitet. In Abhängigkeit vom gewünschten Dispersionsgrad, der Konzentration der Lipidphase und dem Aggregatzustand des Lipids werden als Dispergiersysteme z.B. eingesetzt: Hochdruckhomogenisatoren vom Typ des Kolben-Spalt-Homogenisators (APV Gaulin Systeme, French Press, Avestin), Jet-Stream-Homogenisatoren (z.B. Microfluidizer), Rotor-Stator-Systeme (Ultra-Turrax, Silverson-Homogenisatoren), Ultraschallbad, Ultraschallstab, Ultraschallhomogenisatoren, statische Mischer im Mikro- und Makromaßstab (z. B. Firma Sulzer, Schweiz) sowie Mikromischer (= statische Mikromischer der IMM GmbH, Mainz).

Zur Herstellung der Lipidpartikel durch Präzipitation wird das Lipid in einem Lösungsmittel gelöst und dann mit einem NichtLösungsmittel gemischt. Aufgrund der Löslichkeitsverschlechterung präzipitieren Lipidpartikel. Alternativ kann auch eine Mikroemulsion mit dem geschmolzenen Lipid hergestellt werden. Die bei erhöhter Temperatur erhaltene Mikroemulsion wird dann durch Brechen in eine Makroemulsion überführt, die bei Abkühlen feste Lipidpartikel ausbildet. Brechen der Mikroemulsion kann durch einfaches Abkühlen oder Zugabe von Wasser in die Mikroemulsion erfolgen. Alternativ kann die Mikroemulsion auch in Wasser, bevorzugt kaltes Wasser, gegossen werden.

Die bei der Herstellung der Lipidpartikeldispersion erhaltene Partikelgröße ist eine Funktion von vielen Parametern, z.B.:
- Art des Zerkleinerungsverfahrens
- Tensidkonzentration
- Lipidkonzentration
- Temperatur.

Generell erhält man bei Verfahren mit geringer Leistungsdichte wie einer Mörsermühle Partikel im Größenbereich ca. 50-100 µm. Bei niedriger Tensidkonzentration und hoher Lipidkonzentration können hochtourige Rührer Partikel mit mittlerem Durchmesser im Bereich von wenigen µm bis ca 10-20 µm erzeugen. Bei hoher Tensidkonzentration und gleichzeitig niedriger Lipidkonzentration werden auch Partikel im Nanometerbereich erhalten. Hochfeine Dispersionen mit Teilchengrößen von bis zu ca. 50 nm erzeugt man in der Regel mit Hochdruckhomogenisationsverfahren.

Eine Vielzahl unterschiedlicher Lipide kann zur Herstellung von Lipidpartikeldispersionen eingesetzt werden. Dies sind sowohl chemisch einheitliche Lipide als auch Mischungen derselben. Charakterisiert sind die erfindungsgemäß geeigneten Lipide dadurch, daß sie in der Dispersion im kristallinen Zustand (z.B. β-, βi-Modifikation) oder im flüssig-kristallinen Zustand (α-Modifikation) vorliegen. Es kann auch eine Mischung mehrerer solcher kristallinen oder flüssig-kristallinen Lipide vorliegen. Bei eingesetzten Lipidmischungen können auch flüssige Lipide (z.B. Öle, lipophile Kohlenwasserstoffe, lipophile organische Flüssigkeiten wie Oleylalkohol) den festen Lipiden (z. B. Glyceride, lipophile Kohlenwasserstoffe wie Hartparaffin) zugemischt werden (sog. "lipid blends").

Einsatz finden z. B. folgende Lipide als dispergierte Phase und sie können als individuelle Komponente oder als Mischung angewendet werden: Natürliche oder synthetische Triglyceride bzw. Mischungen derselben, Monoglyceride und Diglyceride, alleine oder Mischungen derselben oder mit z.B. Triglyceriden, selbstemulgierende modifizierte Lipide, natürliche und synthetische Wachse, Fettalkohole, einschließlich ihrer Ester und Ether sowie in Form von Lipidpeptiden, oder beliebige Mischungen derselben. Besonders geeignet sind synthetische Monoglyceride, Diglyceride und Triglyceride als individuelle Substanzen oder als Mischung (z.B. Hartfett, Imwitor 900), Triglyceride (z.B. Glyceroltrilaurat, Glycerolmyristat, Glycerolpalmitat, Glycerolstearat und Glycerolbehenat) und Wachse wie z.B. Cetylpalmitat und weisses Wachs (DAB), außerdem Kohlenwasserstoffe, wie z.B. Hartparaffin.

Als bei Raumtemperatur (20°C) flüssige Lipide können zur Herstellung einer Lipidmischung (lipid blend) z.B. zugemischt werden: mittelkettige Triglyceride (MCT) wie Miglyol (z.B. Miglyol 812, Miglyol 810, Miglyol 840), langkettige Trigylceride (LCT) wie Isopropylmyristat, pflanzliche Öle wie Avocadoöl, Baumwollsamenöl, Distelöl, Erdnußöl, Jojobaöl, Kokosnußöl, Leinöl, Nußöl, Olivenöl, Palmkernöl, Sesamöl, Weizenkeimöl, tierische Öle wie Lebertran, Heilbuttleberöl, Rinderklauenöl einzeln oder in Mischung.

Der Anteil der inneren oder Lipidphase an der Dispersion ist 0,1 % bis 80 % (Gew./Gew. bzw. m/m) und liegt vorzugsweise im Bereich von 1 % bis 40 % (m/m), bezogen auf das Gewicht der Gesamtdispersion) Sollte der Zusatz von dispersionsstabilisierenden Additiven notwendig oder gewünscht sein, z.B. Emulgatoren, um stabile Dispersion zu produzieren zu können, so können diese in Form von reinen Substanzen (z.B. Einzeltensid) oder in Form von Mischungen (Mischemulgatoren, Komplexemulgatoren wie z.B. Lanette N) eingearbeitet sein, um die Partikel zu stabilisieren. Die Menge an solchen Additiven in der Dispersion liegt im Bereich von 0,01 % bis 30 % und vorzugsweise im Bereich von 0,5 % bis 20 %, bezogen auf das Gesamtgewicht der Dispersion.

Zur physikalischen Stabilisierung der Lipidpartikeldispersionen oder zur gezielten Oberflächenmodifikation der Lipidpartikel können die Tenside, Stabilisatoren und Polymere eingesetzt werden, die allgemein aus der Herstellung von Dispersionen bekannt sind. Beispiele dafür sind:
1. sterisch stabilisierende Substanzen wie Poloxamere und Poloxamine (Polyoxyethylen-Polyoxypropylen-Block-Copolymere), ethoxylierte Sorbitanfettsäureester, besonders Polysorbate (z.B. Polysorbat 80 bzw. Tween 80®), ethoxylierte Mono- und Diglyceride, ethoxylierte Lipide, ethoxylierte Fettalkohole oder Fettsäuren, und Ester und Ether von Zuckern oder von Zuckeralkoholen mit Fettsäuren oder Fettalkoholen (z.B. Saccharosestearat, Saccharosedistearat, Saccharoselaurat, Saccharoseoctanoat, Saccharosepalmitat, Saccharosemyristat).
2. geladene ionische Stabilisatoren wie Diacetylphosphate, Phosphatidylglycerin, Lecithine unterschiedlicher Herkunft (z.B. Eilecithin oder Sojalecithin), chemisch modifizierte Lecithine (z.B. hydrierte Lecithine), Phospholipide und Sphingolipide, Mischung von Lecithinen mit Phospholipiden, Sterolen (z.B. Cholesterol und Cholesterol-Derivate wie Stigmasterin) und gesättigte und ungesättigte Fettsäuren, Natriumcholat, Natriumglycocholat, Natriumtaurocholat, Natriumdeoxycholat oder ihre Mischungen, Aminosäuren oder Anti-Flokkulantien, wie z.B. Natriumcitrat, Natriumpyrophosphat, Natriumsorbat, zwitterionische Tenside wie z.B. (3-[(3-Cholamidopropyl)-dimethylammonio]-2-hydroxy-1-propansulfonat)[CHAPSO],(3-[(3-Cholamidopropyl)-dimethyl-ammonio]-1-propansulfonat) [CHAPS] und N-Dodecyl-N,N-dimethyl-3-ammonio-1-propansulfonat, kationische Tenside, insbesondere als Konservierungsmittel eingesetzte Verbindungen, wie z.B. Benzyldimethylhexadecylammoniumchlorid, Methylbenzethoniumchlorid, Benzalkoniumchlorid, Cetylpyridiniumchlorid.
3. viskositätserhoehende Substanzen wie z.B. Cellulose-Ether und Cellulose-Ester (z.B. Methylcellulose, Hydroxyethylcellulose, Hydroxypropylcellulose, Natriumcarboxymethylcellulose), Polyvinylderivate wie Polyvinylalkohol, Polyvinylpyrrolidon, Polyvinylacetat, Alginate, Polyacrylate (z.B.Carbopol), Xanthane und Pektine.

Die geladenen Stabilisatoren sind, wenn notwendig oder gewünscht, vorzugsweise mit 0,01 % bis 20 % (m/m), bezogen auf Gesamtgewicht der Dispersion) und insbesondere in einer Menge von 0,05 % bis zu 10 % in der Lipidpartikeldispersion enthalten.

Viskositätserhöhende Substanzen sind, wenn notwendig oder erwünscht, im ähnlichen Verhältnis in der Formulierung eingearbeitet, vorzugsweise in einer Menge von 0,01-20 % und insbesondere in einer Menge von 0,1 % bis 10 % (m/m) und vorzugsweise im Bereich zwischen 0,5 % und 5 %, bezogen auf Gesamtgewicht der Dispersion).

Als äußere Phase (Dispersionsmedium, kontinuierliche Phase) können Wasser, wäßrige Lösungen oder Flüssigkeiten mischbar mit Wasser, sowie Glycerin oder Polyethylenglykol und ölige Flüssigkeiten wie Miglyole (medium chain triglycerides - MCT) und andere Öle (Rizinus-, Erdnuß-, Soja-, Baumwollsamen-, Raps-, Leinsamen-, Oliven-, Sonnenblumen-, Distelöl eingesetzt werden. Im Prinzip kann jede flüssige Phase eingesetzt werden, sofern sie die Lipidpartikel nicht löst oder anlöst.

Tensidfreie Lipidpartikeldispersionen werden hergestellt durch Dispergierung der Lipidphase in einer wäßrigen Lösung, die eine oder mehrere viskositätserhöhende Substanzen enthält, entweder allein oder in Kombination mit anderen Substanzen, sowie Zucker, Zuckeralkohole, besonders Glukose, Mannose, Trehalose, Mannitol, Sorbitol sowie andere. Desweiteren ist es möglich, eine Kombination der viskositätserhöhenden Stoffe oder die Kombination dieser mit Zuckern oder Zuckeralkoholen, oder in einer weiteren Kombination mit Ladungsstabilisatoren oder Anti-Flokkulantien zu gebrauchen.

Die Partikelbildung zur Erzielung einer engen Partikelgrößenverteilung und unter Minimierung von Partikelaggregaten kann durch weitere Zusätze gefördert werden. Derartige Zusätze sind Substanzen, die den pH-Wert verschieben (z. B. Erhöhung des Zetapotentials, Beeinflussung der Tensidstruktur wie Dissoziationsgrad) oder die Stabilität der Lipidpartikeldispersion über andere Mechanismen erhöhen, z. B. über Beeinflussung der Wasserstruktur (z. B. Zugabe von Elektrolyten) oder durch Effekte auf die stabilisierende Tensidschicht (z. B. Glukose bei Lecithin).

Die zusätzliche Beladung der Lipidpartikel mit UV-blockierenden Substanzen, Antioxidantien wie Tocopherol und hautpflegenden Substanzen (z.B. Retinol und seine Derivate, Harnstoff) - hier alle zusammengefaßt als "Wirkstoffe" - kann auf verschiedenen Wegen, einzeln oder in Kombination erfolgen. Der oder die Wirkstoffe sind in den Lipidteilchen gelöst, lösungsvermittelt (z.B. mit Tensiden oder Cyclodextrinen) oder dispergiert. Ferner können sie an deren Oberfläche adsorbiert sein. Aufgrund des Feststoffcharakters der Partikelmatrix können auch hydrophile Wirkstoffe in Form einer wäßrigen Wirkstofflösung in die Lipidphase eingearbeitet werden. Nach dieser Einarbeitung und der anschließenden Dispergierung des Lipids in dem wäßrigen Dispersionsmedium entsteht ein System W/F/W, d.h. Wasser in Fett in Wasser. Der Lipidkern schließt hierbei die wäßrige Wirkstofflösung aufgrund seines festen Aggregatzustandes besser ein als es bei vergleichbaren multiplen Emulsionen Wasser in Öl in Wasser (W/Ö/W) möglich ist.

Die Einarbeitung des oder der Wirkstoffe kann nach verschiedenen Methoden erfolgen. Beispielhaft seien genannt:
1. Lösen des Wirkstoffs in der inneren (z.B. geschmolzenen) Phase.
2. Lösen des Wirkstoffs in einem mit der inneren Phase mischbaren Lösungsmittel und Zugabe dieser Wirkstofflösung zur inneren Phase. Anschließend wird gegebenenfalls das Lösungsmittel teilweise oder vollständig entfernt.
3. Dispergieren des Wirkstoffs in der inneren Phase (z.B. durch Dispergieren eines Feststoffs wie Titandioxid oder gezielte Präzipitation in der inneren Phase).
4. Lösen des Wirkstoffs in der äußeren, wäßrigen Phase (z.B. amphiphile Substanzen) und Einbindung des Wirkstoffs in einen die Lipidpartikel stabilisierenden Tensidfilm während der Herstellung.
5. Adsorption des Wirkstoffs an der Teilchenoberfläche.
6. Lösen des Wirkstoffs in der Lipidphase mittels eines Lösungsvermittlers (z.B. eines Blockcopolymeren, Sorbitanfettsäureesters, Cyclodextrins), anschließende Dispergierung der Lipidphase zur Herstellung der Vordispersion. Der Wirkstoff liegt dann in den Partikeln als feste Lösung vor.
7. Einarbeiten von wäßrigen Wirkstofflösungen in die Lipidphase und anschließende Dispergierung der Lipidphase zur Herstellung der Vordispersion, so daß ein System W/F/W entsteht, das den multiplen Emulsionen analog ist.
8. Dispergieren des Wirkstoffes in der geschmolzenen Lipidphase über einen Quellungs- oder Gelbildungsprozeß (z.B. Aerosil als Oleogelbildner in geschmolzenem Lipid).

Als molekulare UV-Blocker können erfindungsgemäß unter anderem eingesetzt werden: Benzophenon und seine Derivate wie 4-Phenylbenzophenon, 2-Hydroxy-4-n-octyloxybenzophenon, 2-Hydroxy-4-methoxybenzophenon, 2,2'-Dihydroxy-4,4'-dimethoxybenzophenon, Sulisobenzonum, Benzimidazolderivate wie Phenylbenzimidazolsulfonsäure, Campherderivate wie 3-Benzylidencampher, 3-(4-Methyl-benzyliden)campher, Terephthalylidendicamphersulfonsäure, Dibenzoylmethane wie 4-Isopropyl-dibenzoylmethan, 4-tert.-Butyl-4'-methoxy-dibenzoylmethan, Zimtsäureester wie p-Methoxyzimtsäure-2-ethylhexylester, p-Methoxyzimtsäureisoamylester, p-Methoxyzimtsäureoctylester, p-Methoxyzimtsäurepropylester p-Aminobenzoesäure (PABA) und seine Derivate wie p-Aminobenzoesäureglycerolester, Butyl-PABA, Octyl-dimethyl-PABA, oder weitere Substanzen wie 2-Ethylhexylsalicylat, Homosalat, Mexoryl® SX, Mexoryl® XL Octylsalicylat, Octyltriazon, Oxybenzon, einzeln oder in Mischung.

Als anorganische Pigmente oder organische Pigmente (partikuläre UV-Blocker) können erfindungsgemäß unter anderem eingesetzt werden: Bariumsulfat, Bentonite, Calciumcarbonat, Calciumsulfat, Eisen-(III)-oxide, Eisenoxidhydrat, Kaolin, Kohlenschwarz, Kupferoxid, Magnesiumoxid, Silber, Siliziumdioxid (z.B. Aerosile), Syloid, hydrophobes alkyliertes Siliziumdioxid (z.B. Aerosil R972), Talcum, Titandioxid, Wismutoxychlorid, Zinkoxid, Zinkstearat, Melanin einzeln oder in Mischung.

Als antioxidativ wirkende Substanzen können erfindungsgemäß unter anderem eingesetzt werden: Retinol, Retinolderivate wie Retinolpalmitat, Retinolacetat, Vitamin E, Vitamin E-Derivate wie Vitamin E-Acetat, Vitamin E-Linoleat, Vitamin E-Nicotinat, Vitamin E-Palmitat, Vitamin E-POE(22)succinat, Vitamin C, Vitamin C-Derivate wie z.B. Vitamin C-Palmitat, Magnesiumascorbat, Magnesiumphosphat, Aescin, Butylhydroxyanisol (BHA), Butylhydroxytoluol (BHT), Cystein, Dilaurylthiodipropionat, Dodecylgallat, Kaffeesäure Propylgallat, einzeln oder in Mischung.

Als hautpflegende Substanzen und/oder feuchtigkeitserhöhende Substanzen können erfindungsgemäß unter anderem eingesetzt werden: Aminosäurederivate wie Arginin-Pyroglutaminat, Glutaminsäure, Lysin-Pyroglutaminat, Glucose, Glycerol, Harnstoff, Mucopolysaccharide wie Hyaluronsäure, Natriumlactat, Natrium-Pyrrolidoncarbonsäure, Propylenglycol, Retinole, Vitamin A und seine Derivate, Saccharoseglutamat, Allantoin, Biotin, Bisabolol, Cholesterol, Collagen und seine Derivate, Elastin, Glycoproteine, Hyaluronsäure und seine Derivate, Keratin und seine Derivate, Lecithin, Linolsäure, Linolensäure, Milchproteine, Niacinamid,Panthenol und seine Derivate, Riboflavin, Schwefel, Harnstoff, Sojabohnenöl, Tocopherol und seine Derivate, einzeln oder in Mischung.

Zur Herstellung und Charakterisierung der Lipidpartikel in den Beispielen wurden folgende Geräte eingesetzt: Ultra-Turrax T25, Janke und Kunkel, Staufen, mit Dispergierwerkzeug S25 KR; Micron Lab 40, APV Homogeniser, Lübeck; Coulter LS230, Coulter Electronics, Krefeld; Zetasizer 4, Malvern Instruments, Essen; Uvikon 940 Spectrophotometer, Kontron, Neufahn; Rasterelektronenmikroskop S360, Cambridge Instruments, England.

Die verwendeten Lipide, Tenside und UV-Blocker waren: Precifac ATO, Gattefossé, Frankreich; Tego Care 450, Th. Goldschmidt, Essen; Stearylalkohol, Fluka, Neu-Ulm; Tween 80, Merck, Darmstadt; Eusolex 4360, Merck, Darmstadt

Die Partikel verstärken die natürliche Hautbarriere, indem sie einen geschlossenen Lipidfilm beim Ausstreichen bilden (Beispiel 18). Im Gegensatz zur bekannten löcherigen, porösen Filmbildung mit dichter Kugelpackung wurde beim Aufstreichen der Lipidpartikeldispersion zur Herstellung von Filmen die Ausbildung eines geschlossenen Lipidfilmes beobachtet und mit Elektronenmikroskopie nachgewiesen. Eine beschädigte natürliche Lipidbarriere der Haut kann dadurch repariert bzw. ersetzt werden.

Zur Herstellung von Sonnenschutzmitteln können Lipidpartikeldispersionen mit einem höheren Lipidgehalt (z.B. > ca. 40 %), bezogen auf das Gesamtgewicht der Dispersion , hergestellt werden (d.h. beispielsweise > 40 g Lipid in 100 g Dispersion), die aufgrund der hohen Feststoffkonzentration in der Regel eine ausreichend hohe Konsistenz besitzen, so daß sie sich zum Auftragen auf die Haut eignen. Bei niedriger konzentrierten Lipidpartikeldispersionen kann es erforderlich sein die Viskosität der äußeren Phase zu erhöhen, z.B. durch Zusatz eines Gelbildners. Die Wahl des Gelbildners erfolgt in Abhängigkeit von der chemischen Natur der äußeren Phase (z.B. Hydroxyethylcellulose bei Wasser, Aerosil bei Wasser oder Öl etc.). Alternativ können die erfindungsgemäßen Lipidpartikel Lotionen (z.B. O/W Emulsionen), Cremes oder Salben zugesetzt bzw. in diese eingearbeitet werden. der Zusatz von grobdispersen Lipidpartikeln kann dadurch erfolgen, daß das Lipidpulver in diese Systeme eingerührt wird. Hochfeine Lipid-partikel (z.B. im Nanometerbereich) können als höher konzentrierte Dispersion zugemischt werden. Alternativ können Lipid-partikeldispersionen direkt bei der Herstellung von Lotionen und Cremes eingearbeitet werden, indem man einen Teil der Wasserphase durch eine ausreichend hoch konzentrierte Lipidpartikeldispersion ersetzt.

### Beispiele

### Beispiel 1:

### UV-blockierende Wirkung von Cetylpalmitat-Partikeln im Vergleich zu Miglyol-Emulsionen

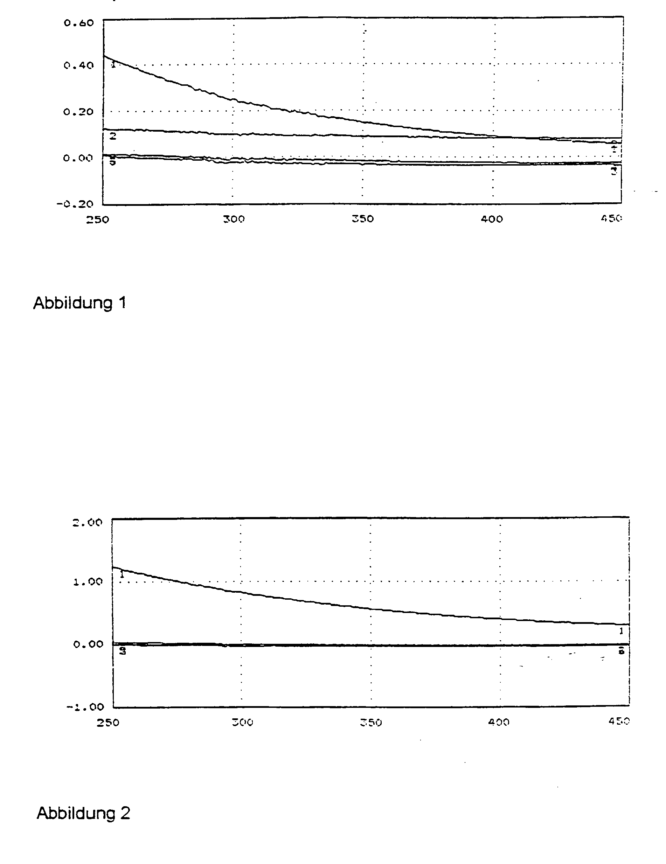

Es wurde eine Lipidpartikel-Dispersion bestehend aus 10 % (m/m) Cetylpalmitat, 1,2 % (m/m) Polyglycerol-methyl-glucosedistearat (Tego Care 450) und Wasser durch Hochdruckhomogenisation hergestellt. Die Mischung von Lipid und Emulgator wurde bei 75°C geschmolzen und in der wäßrigen Lösung mit einem Ultra-Turrax T25 mit Dispergierwerkzeug S25, Janke und Kunkel, dispergiert (8000 rpm, für 1 Minute). Die erhaltene Rohemulsion wurde dann mit einem APV Gaulin LAB 40 Homogenisator bei 500 bar mit 3 Zyklen bei 75°C homogenisiert. Es entstanden Lipidpartikel mit einem PCS-Durchmesser von 221 nm und einem Polydispersitätsindex von 0,06. Als Vergleich wurde ein Emulsionssystem hergestellt, wobei die 10 % Cetylpalmitat durch 10 % Miglyol 812 ersetzt wurden. Herstellparameter war Dispergierung mit dem Ultra-Turrax (8000rpm für 1 Minute). Die UV-blockierende Wirkung wurde mit einem Uvikon 940 Spektrophotometer, Kontron, in dem Wellenlängenbereich von 250-450 nm untersucht. Lipidpartikel-Dispersion und Emulsion wurden hierfür verdünnt (5 µL in 1 mL Wasser), gemessen wurde gegen Wasser. Die Emulsion zeigte über den gemessenen Bereich eine konstante Absorption von ca. 0,15, die Lipidpartikel-Dispersion einen Anstieg der Absorption von 0,1 bei 450 nm auf 0,45 bei 250 nm. Messungen einer reinen Lipidlösung (in 96 % Ethanol) bzw. wäßriger Tensidlösung gleicher Konzentration absorbierten über den gesamten Meßbereich nicht (Abb. 1).

### Beispiel 2:

### UV-blockierende Wirkung von Stearylalkohol-Lipidpartikeln

Lipidpartikel und Emulsion wurden wie in Beispiel 1 hergestellt, das Tensid war 1,2 % Polysorbat 80 (Tween 80). Im Spektrophotometer zeigte die Emulsion aus Miglyol über den gesamten Bereich lediglich einen Absorptionswert von 0 - 0,05 (d.h. dies ist nahe dem Grundrauschen des Gerätes), die Lipidpartikel aus Stearylalkohol besaßen eine ansteigende Absorption von 0,3 bei 450 nm bis auf 1,3 bei 250 nm. Messungen einer reinen Lipidlösung (in 96 % Ethanol) bzw. wäßriger Tensidlösung gleicher Konzentration absorbierten über den gesamten Meßbereich nicht (Abb. 2).

### Beispiel 3:

### UV-blockierende Wirkung von Lipidpartikeln nach Ausbildung eines Films

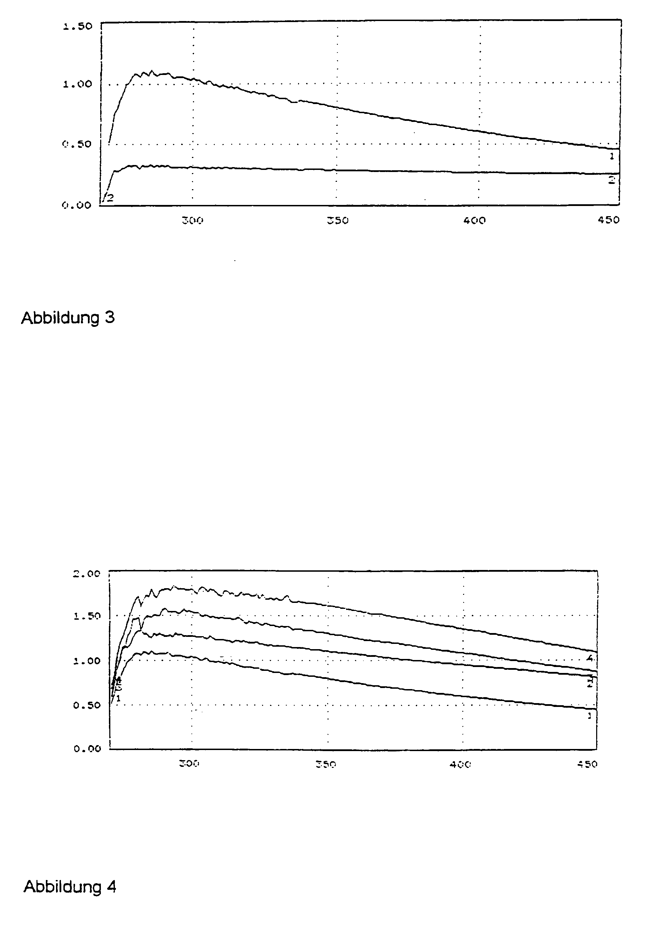

Es wurde eine Lipidpartikel-Dispersion mit Cetylpalmitat und dem Tensid Polyglycerol-methyl-glucosedistearat (Tego Care 450) nach Beispiel 1 hergestellt. Als Vergleich diente die Emulsion mit Miglyol und dem Tensid TegoCare hergestellt wie beschrieben in Beispiel 1. Die beiden Formulierungen wurden auf ein auf eine Quarz-Meßküvette aufgeklebtes Transpore™ Tape aufgetragen (50 µL auf 4,5 cm² Transpore™ Tape), verstrichen und sofort vermessen. Die UV-blockierende Wirkung der gebildeten Filme wurde im Spektrophotometer untersucht, wobei als Referenz unbeschichtetes Transpore Tape auf eine Küvette aufgeklebt wurde. Für den Emulsionsfilm ergab sich über den gemessenen Bereich (450-250 nm) eine relativ konstante Absorption von 0,25-0,30; die Absorption der Lipidpartikel stieg von 0,45 bei 450 nm auf 1,1 bei 280 nm an (Abb. 3).

### Beispiel 4:

### Erhöhung der Absorption in Abhängigkeit der Lipidkonzentration

Lipidpartikel aus Cetylpalmitat stabilisiert mit Tego Care wurden bei unterschiedlichen Lipidkonzentrationen produziert. Die Lipidkonzentrationen betrugen 10 %, 20 %, 30 % und 40 % bei proportionaler Tego Care Konzentration von 1,2 %, 2,4 %, 3,6 %, und 4,8 %. Die korrespondierenden Laser-Diffraktometrie LD-50 %-Durchmesser waren 138 nm, 214 nm, 142 nm und 178 nm mit ansteigender Lipidkonzentration. Die Absorption der analog Beispiel 3 auf Transpore™ Tape aufgetragenen Filme stieg konzentrationsabhängig an (Abb. 4).

| Lipidkonzentration | Absorption bei 450 nm | Absorption bei 280 nm |
|---|---|---|
| 10 % | 0,45 | 1,1 |
| 20 % | 0,8 | 1,33 |
| 30 % | 0,9 | 1,58 |
| 40 % | 1,1 | 1,8 |

### Beispiel 5:

### UV-blockierende Wirkung als Funktion der Partikelgröße

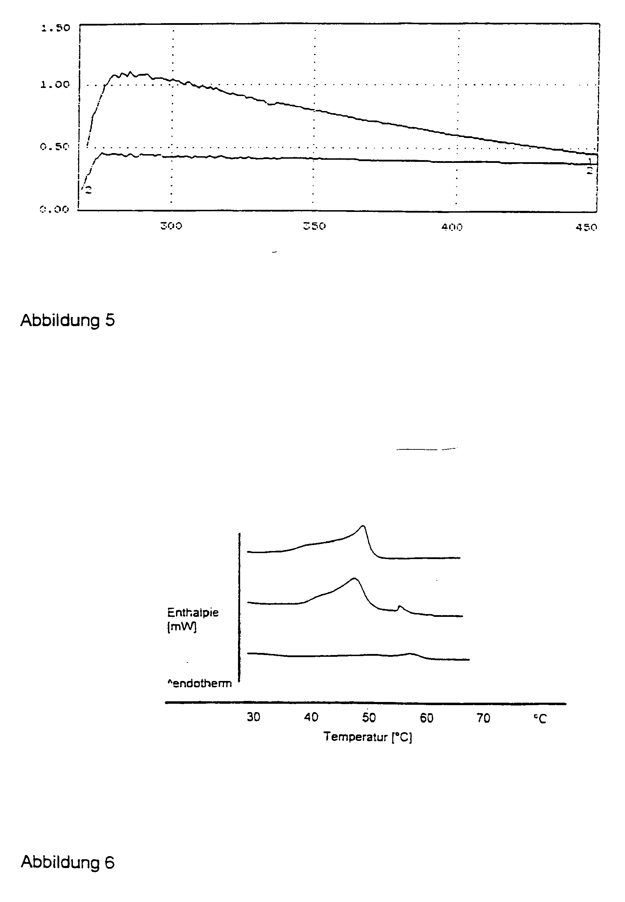

Lipidpartikel wurden hergestellt analog Beispiel 1. Die Zusammensetzung betrug 10 % Lipid, 1,2 % Tensid und Wasser. Herstellung der Lipide erfolgte durch Dispergierung im geschmolzenen Zustand (75 °C) mit einem hochtourigen Ultra-Turrax-Rührer (8000 rpm für 5 Minuten) und alternativ mit Hochdruckhomogenisation (Bedingungen wie in Beispiel 1). Die Partikelgröße mit dem Rührer betrug 4,6 µm (d50 % - Durchmesser 50 %) nicht erfindungsgemäß, die Partikelgröße nach Hochdruckhomogenisation 138 nm (d50 %). Beide Lipidpartikel-Dispersionen wurden wie in Beispiel 3 beschrieben auf Transpore™ Tape aufgebracht und nach Trocknung bei Raumtemperatur sofort im UV-Spektrophotometer vermessen. Die Absorption betrug über den gesamten Meßbereich etwa 0,45 für die Lipid-Mikropartikel und stieg für die durch Hochdruckhomogenisation hergestellten LipidNanopartikel von 0,45 bei 450 nm auf 1,1 bei 280 nm an (Abb. 5).

### Beispiel 6:

### Stabiltität der festen Lipidpartikel nach Einarbeitung in eine Creme

Lipidpartikel folgender Zusammensetzung wurden hergestellt: 10 % Cetylpalmitat, 1,2 % Polyglycerol-methyl-glucosedistearat (Tego Care 450) und Wasser. Die Mischung von Lipid und Emulgator wurde bei 75 °C geschmolzen und in der wäßrigen Lösung mit einem Ultra-Turrax T25 mit Dispergierwerkzeug S25, Janke und Kunkel dispergiert (8000 rpm, für 1 Minute). Die erhaltene Rohemulsion wurde dann mit einem APV Gaulin LAB 40 Homogenisator bei 500 bar mit 3 Zyklen bei 75 °C homogenisiert. Es entstanden Lipidpartikel mit einem PCS-Durchmesser von 220 nm und einem Polydispersitätsindex von 0,06. Die Lipidpartikel wurden im Verhältnis 1 : 1 mit einer im Handel erhältlichen O/W-Emulsion gemischt. Die Mischung erfolgte durch Rühren in einer Fantaschale mit Pistill. Die Integrität der Partikel wurde mit Differential Scanning Calorimetry (DSC) bestimmt. Der Schmelzpeak der Lipidpartikel-Dispersion betrug 16,8 J/g, nach Einarbeitung einer äquivalenten Menge an Lipidpartikel-Dispersion in die Creme betrug der Schmelzpeak in der Creme 16,6 J/g. Die Partikel waren physikalisch stabil über 6 Monate. Nach 6 Monaten Lagerung bei 20 °C betrug der Schmelzpeak 16,2 J/g und war nicht signifikant unterschiedlich vom Ausgangswert (Abb. 6).

### Beispiel 7:

### Gleichmäßigkeit der auf Transpore™ Tape aufgetragenen Filme

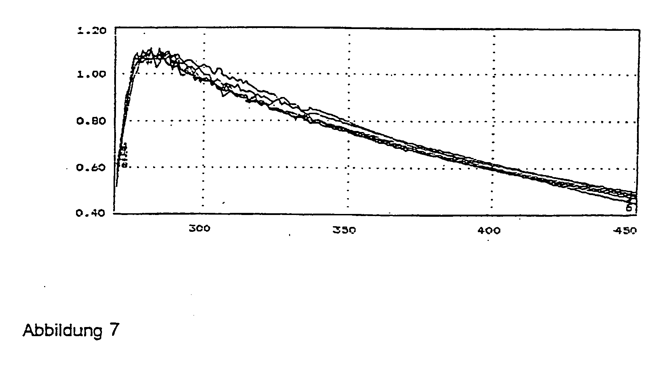

Eine Lipidpartikeldispersion aus 10 % Cetylpalmitat, 1,2 % Polyglycerol-methyl-glucosedistearat (Tego Care 450) und Wasser wurde hergestellt. 50 µl dieser Dispersion wurden analog Beispiel 3 gleichmäßig auf eine Fläche von 4,5cm² einer mit Transpore™ Tape beklebten Quarzküvette aufgetragen und über den Wellenlängenbereich von 450 bis 250 nm vermessen. Dabei wurde die Küvette in unterschiedlichen Stellungen im Halter befestigt und somit der Film über einer Länge von 8mm vermessen. Die Absorptionswerte schwanken kaum, so daß Filmgleichmäßigkeit gegeben ist (Abb. 7).

### Beispiel 8:

### UV-blockierende Wirkung von UV-Blocker enthaltenden Lipidpartikeln nach Ausbildung eines Films

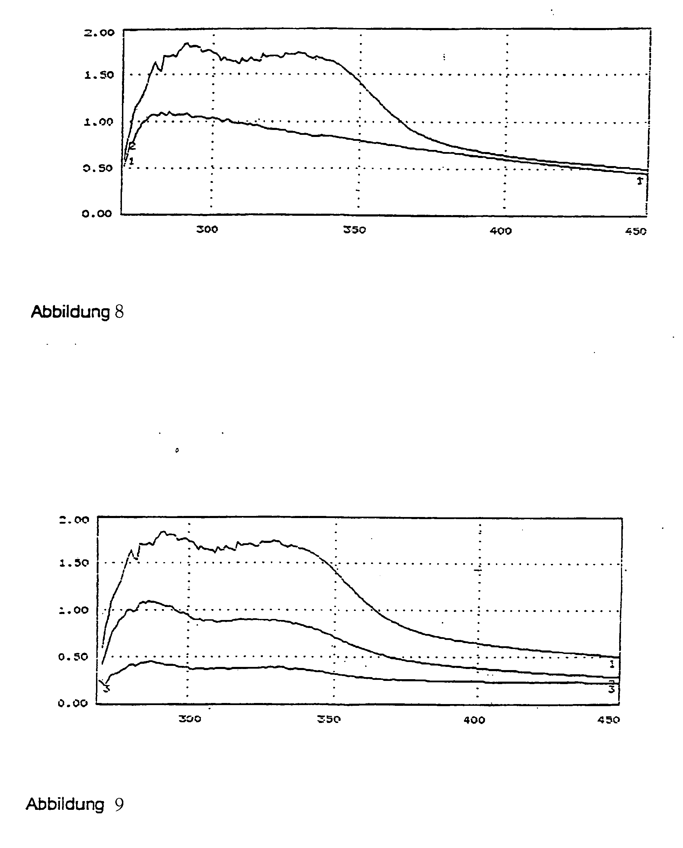

Es wurde eine Lipidpartikel-Dispersion mit Cetylpalmitat und dem Tensid Polyglycerol-methyl-glucosedistearat (Tego Care 450) nach Beispiel 1 hergestellt, wobei der lipophile Breitbandfilter 2-Hydroxy-4-methoxy-benzophenon (Eusolex 4360) in einer Konzentration von 10 % bezogen auf das Lipid (entspricht 1 % bezogen auf den Gesamtansatz) mit der Lipidphase aufgeschmolzen und so eingearbeitet wurde. Als Vergleich diente die reine Lipidpartikeldispersion, hergestellt wie beschrieben in Beispiel 1. Die beiden Formulierungen wurden auf ein auf eine Quarz-Meßküvette aufgeklebtes Transpore™ Tape aufgetragen (50 µL auf 4,5 cm² Transpore™ Tape), verstrichen und sofort vermessen. Die UV-blockierende Wirkung der gebildeten Filme wurde im Spektrophotometer untersucht, wobei als Referenz unbeschichteter Transpore™ Tape auf eine Küvette aufgeklebt wurde. Die UV-Blocker enthaltende Dispersion zeigte im Bereich unterhalb 380 nm eine deutlich höhere Absorption mit dem für Eusolex 4360 typischen Verlauf (Peaks bei ca. 335 und 290 nm) als die reinen Lipidpartikel (Abb. 8).

### Beispiel 9:

### Erhöhung der Absorption in Abhängigkeit der UV-Blocker-Konzentration

Lipidpartikel-Dispersionen mit 10 % Cetylpalmitat, 1,2 % Polyglycerol-methyl-glucosedistearat (Tego Care 450) und Wasser wurden nach Beispiel 1 hergestellt, wobei bezogen auf das Lipid 10 %, 5 % und 1 % 2-Hydroxy-4-methoxy-benzophenon (Eusolex 4360) analog Beispiel 8 eingearbeitet wurde. Die Dispersionen wurden nach Beispiel 3 auf Transpore™ Tape aufgetragen und vermessen. Die Absorption war konzentrationsabhängig, wenn auch nicht proportional (Abb. 9).

### Beispiel 10:

### UV-blockierende Wirkung von UV-Blocker enthaltenden Lipidpartikeln nach Ausbildung eines Films

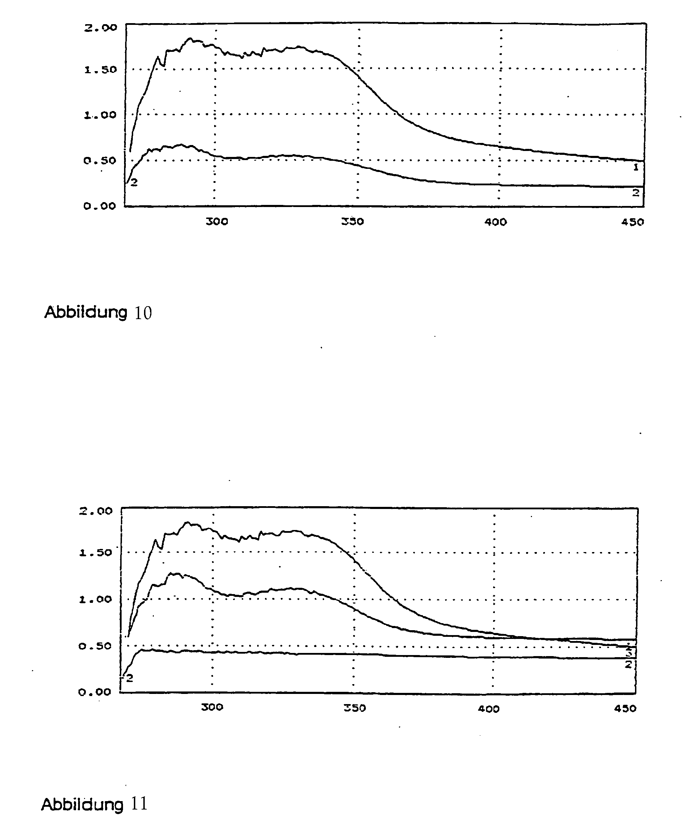

Es wurde eine Lipidpartikel-Dispersion mit Cetylpalmitat, dem Tensid Polyglycerol-methyl-glucosedistearat (Tego Care 450) und 10 % 2-Hydroxy-4-methoxy-benzophenon (Eusolex 4360) bezogen auf den Lipidgehalt, nach Beispiel 8 hergestellt. Als Vergleich diente die Emulsion mit Miglyol und dem Tensid TegoCare, hergestellt wie beschrieben in Beispiel 1, wobei hier ebenfalls 10 % Eusolex 4360 bezogen auf den Miglyolgehalt eingearbeitet wurde. Die beiden Formulierungen wurden auf ein auf eine Quarz-Meßküvette aufgeklebtes Transpore™ Tape aufgetragen (50 µL auf 4,5 cm² Transpore Tape), verstrichen und sofort vermessen. Die UV-blockierende Wirkung der gebildeten Filme wurde im Spektrophotömeter untersucht, wobei als Referenz unbeschichteter Transpore™ Tape auf eine Küvette aufgeklebt wurde. Für den Emulsionsfilm ergab sich über den gemessenen Bereich (450-250 nm) eine Absorption, die deutlich unterhalb der Absorption der Lipidpartikeldispersion lag (Abb. 10).

### Beispiel 11:

### UV-blockierende Wirkung nach Einarbeitung eines UV-Blockers als Funktion der Partikelgröße

Lipidpartikel wurden hergestellt analog Beispiel 8. Die Zusammensetzung betrug 10 % Lipid, 1,2 % Tensid, 10 % UV-Blocker bezogen auf den Lipidgehalt und Wasser. Herstellung der Lipide erfolgte durch Dispergierung im geschmolzenen Zustand (75 °C) mit einem hochtourigen Ultra-Turrax-Rührer (8000 Umdrehungen pro Minute, 5 Minuten) und alternativ mit Hochdruckhomogenisation (Bedingungen wie in Beispiel 1). Die Partikelgröße mit dem Rührer betrug 12 µm (d50 %), die Partikelgröße nach Hochdruckhomogenisation 138 nm (d50 %). Beide Lipidpartikel-Dispersionen wurden wie in Beispiel 3 beschrieben auf Transpore™ Tape aufgebracht und nach Trocknung bei Raumtemperatur sofort im UV-Spektrophotometer vermessen. Die Absorption der Mikropartikel lag im ganzen UV-Bereich deutlich unter der Absorption der Nanopartikel (Abb. 11).

### Beispiel 12:

### UV-blockierende Wirkung nach Einarbeitung eines UV-Blockers und eines hautpflegenden Arzneistoffs

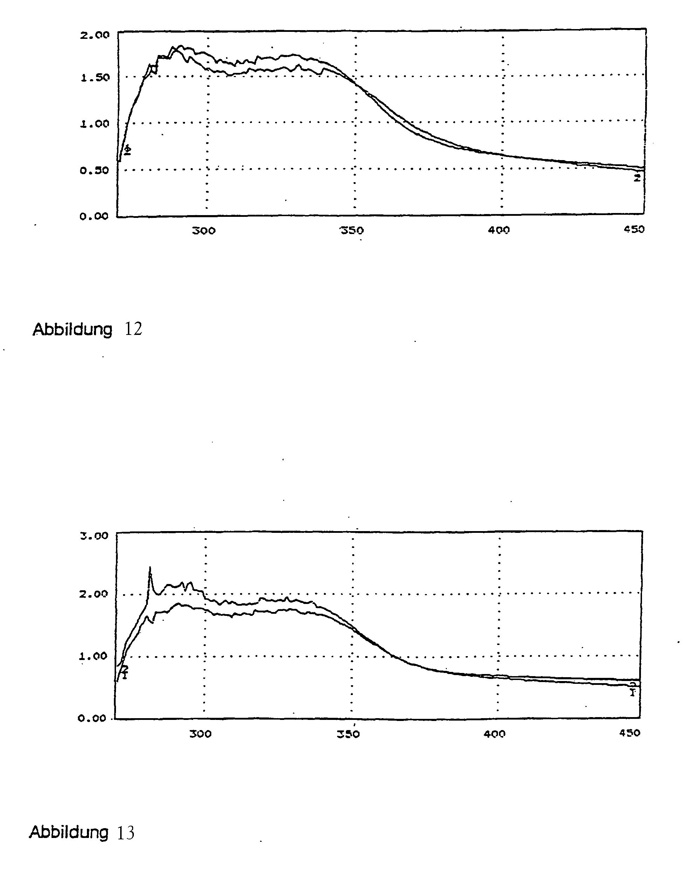

Lipidpartikel aus 10 % Cetylpalmitat, 1,2 % Polyglycerol-methyl-glucosedistearat (Tego Care 450) und 10 % 2-Hydroxy-4-methoxybenzophenon (Eusolex 4360) (letzteres bezogen auf den Lipidgehalt) wurden nach Beispiel 8 hergestellt, wobei als weiterer Bestandteil Retinolpalmitat in einer Konzentration von 0,2 % bezogen auf den Gesamtansatz durch gemeinsames Aufschmelzen mit der Lipidphase eingearbeitet wurde. Die Lipidpartikeldispersion wurde analog Beispiel 3 als Film vermessen, wobei die nur UV-Blocker enthaltende Lipidpartikeldispersion als Referenz diente. Über den gesamten Meßbereich zeigten die Vitamin A Palmitatenthaltenden Lipidpartikel nur geringfügige Abweichungen zur Referenz (Abb. 12).

### Beispiel 13:

### UV-blockierende Wirkung von Lipidpartikeln nach Einarbeitung eines UV-Blockers und eines Antioxidans

Lipidpartikel aus 10 % Cetylpalmitat, 1,2 % Polyglycerol-methyl-glucosedistearat (Tego Care 450) und 10 % 2-Hydroxy-4-methoxybenzophenon (Eusolex 4360) (letzteres bezogen auf den Lipidgehalt) wurden nach Beispiel 8 hergestellt, wobei als weiterer Bestandteil Tocopherol in einer Konzentration von 2 % bezogen auf den Gesamtansatz durch gemeinsames Aufschmelzen mit der Lipidphase eingearbeitet wurde. Die Lipidpartikeldispersion wurde analog Beispiel 3 als Film vermessen, wobei die nur UV-Blocker enthaltende Lipidpartikeldispersion als Referenz diente. Über den gesamten Meßbereich zeigten die Vitamin E-enthaltenden Lipid-partikel nur geringfügige Abweichungen zur Referenz (Abb. 13). **Beispiel 14:**

### UV-blockierende Wirkung von Aerosil enthaltenden Lipidpartikeln nach Ausbildung eines Films

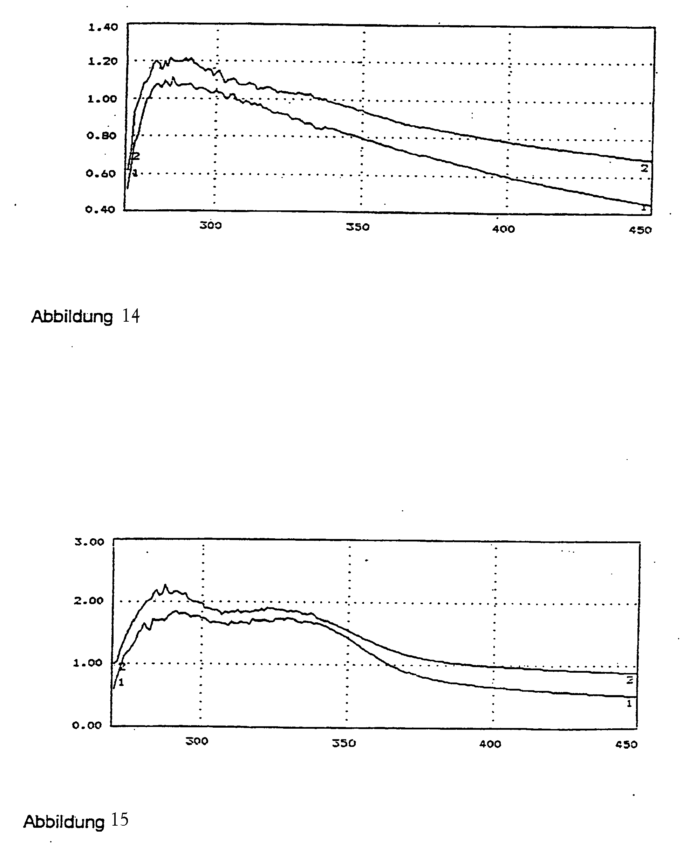

Es wurde eine Lipidpartikel-Dispersion mit Cetylpalmitat und dem Tensid Polyglycerol-methyl-glucosedistearat (Tego Care 450) nach Beispiel 1 hergestellt, wobei hochdisperses Siliziumdioxid (Aerosil 200) in einer Konzentration von 5 % bezogen auf den Lipidgehalt gemeinsam mit der Lipidphase aufgeschmolzen wurde, 5 Minuten bei 75 °C quellen gelassen wurde und so eingearbeitet wurde. Als Vergleich diente die reine Lipidpartikeldispersion, hergestellt wie beschrieben in Beispiel 1. Die beiden Formulierungen wurden auf ein auf eine Quarz-Meßküvette aufgeklebtes Transpore™ Tape aufgetragen (50 µL auf 4,5 cm² Transpore™ Tape), verstrichen und sofort vermessen. Die UV-blockierende Wirkung der gebildeten Filme wurde im Spektrophotometer untersucht, wobei als Referenz unbeschichteter Transpore™ Tape auf eine Küvette aufgeklebt wurde. Die Lipidpartikeldispersion, die zusätzlich Aerosil enthielt, absorbierte erkennbar stärker als die Vergleichsformulierung (Abb. 14).

### Beispiel 15:

### UV-blockierende Wirkung von Aerosil und UV-Blocker enthaltenden Lipidpartikeln nach Ausbildung eines Films

Eine Lipidpartikel-Dispersion mit dem UV-Blocker 2-Hydroxy-4-methoxy-benzophenon (Eusolex 4360) (10 % bezogen auf Lipid) wurde analog Beispiel 8 hergestellt, wobei zusätzlich 5 % Aerosil 200 bezogen auf das Lipid wie in Beispiel 14 eingearbeitet wurden. Als Vergleich diente die gleiche Formulierung jedoch ohne Aerosil 200. Die beiden Formulierungen wurden auf ein auf eine Quarz-Meßküvette aufgeklebtes Transpore™ Tape aufgetragen (50 µL auf 4,5 cm² Transpore™ Tape), verstrichen und sofort vermessen. Die UV-blockierende Wirkung der gebildeten Filme wurde im Spektrophotometer untersucht, wobei als Referenz unbeschichteter Transpore™ Tape auf eine Küvette aufgeklebt wurde. Die Lipidpartikeldispersion, die zusätzlich Aerosil enthielt, absorbierte stärker als die Vergleichsformulierung (Abb. 15).

### Beispiel 16:

### Synergismus von festen Lipidnanopartikeln und UV-Blockern als Filme

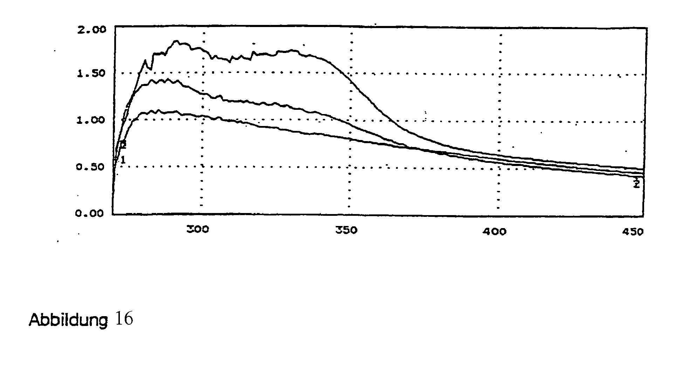

Rechnerisch wurde die Eigenabsorption des UV-Blockers 2-Hydroxy-4-methoxy-benzophenon (Eusolex 4360) ermittelt, indem die Absorption der Miglyol - Tego Care Emulsion (s. Beispiel 3) von der Absorption der Eusolex 4360-enthaltenden Miglyol-Tego Care Emulsion (s. Beispiel 10) über den Bereich von 450 bis 250 nm subtrahiert wurde. Diese Werte wurden zu der Absorption von reinen Lipidpartikeln (s. Beispiel 3) addiert, um die theoretische Absorption von UV-Blocker enthaltenden Lipidpartikeln zu erhalten. Vergleicht man die theoretische jedoch mit der praktisch gemessenen Absorption von Lipidpartikeln, die Eusolex 4360 enthalten, so stellt man einen Synergismus fest, da die theoretische Absorption über den gesamten UV-Bereich deutlich niedriger ist (Abb. 16).

### Beispiel 17:

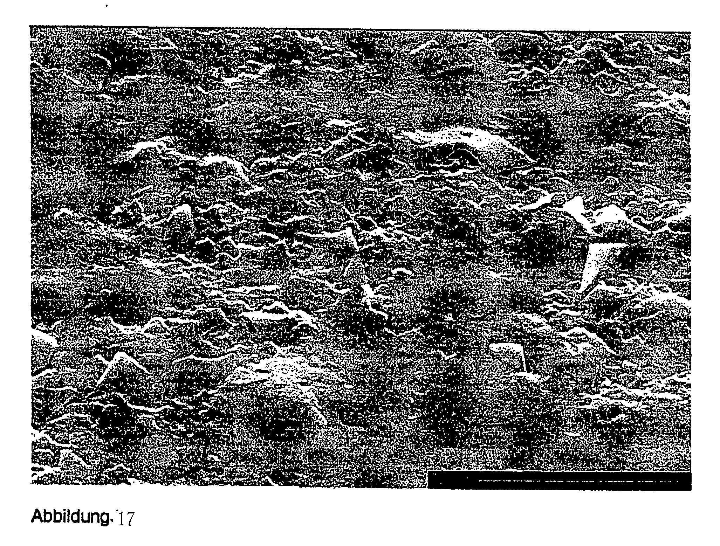

Eine Lipidpartikeldispersion bestehend aus 10 % Cetylpalmitat, 1,2 % Tego Care 450 und Wasser, hergestellt analog Beispiel 1 wurde auf beidseitig klebenden Tesa Film aufgetragen, über Nacht eintrocknen gelassen und rasterelektronenmikroskopisch mit einem S 360, Cambridge Instruments Mikroskop untersucht. Hierbei wurde ein geschlossener Lipidfilm detektiert (Abb. 17).

## Patentansprüche

1. Verwendung von festen, polymorphen, kristallinen oder teilkristallinen Lipidpartikeln oder einer Zusammensetzung, die feste, polymorphe kristalline oder teilkristalline Lipidpartikel, gegebenenfalls als feste innere Phase (Lipidphase) dispergiert in einer äußeren flüssigen Phase umfaßt, als mittel zum Schutz vor zur Strahlung wobei die Lipidpartikel eine Größe, ausgedrückt als Mittelwert der Hauptpopulation, im Bereich von 10 nm bis 1000 nm aufweisen und während der Aufheizphase in der Wärmekalorimetrie (DSC - Differential Scanning Calorimetry) einen endothermen Peak oberhalb 20 °C zeigen, zur Herstellung von Mitteln mit UV-Strahlung absorbierender und/oder reflektierender Wirkung zur Anwendung auf der Haut, Schleimhäuten, Haaren und Kopfhaut zum Schutz vor gesundheitsschädlicher UV-Strahlung und Stärkung der natürlichen Hautbarriere.

2. Verwendung nach Anspruch 1 , **dadurch gekennzeichnet, daß** eine Zusammensetzung eingesetzt wird, die außerdem einen oder mehrere molekulare und/oder partikuläre UV-Blocker enthält, die in dem Lipidmatrixmaterial gelöst und/oder dispergiert und/oder an der Oberfläche der Lipidpartikel adsorbiert sind, wobei sie als molekularen UV-Blocker eine oder mehrere Substanzen ausgewählt aus Benzophenon und seinen Derivaten, insbesondere 4-Phenylbenzophenon, 2-Hydroxy-4-n-octyloxybenzophenon, 2-Hydroxy-4-methoxybenzophenon, 2,2'-Dihydroxy-4,4'-dimethoxybenzophenon, Sulisobenzonum, Benzimidazolderivaten, insbesondere Phenylbenzimidazolsulfonsäure, Campherderivaten, insbesondere 3-Benzylidencampher, 3-(4-Methylbenzyliden)campher, Terephthalylidendicamphersulfonsäure, Dibenzoylmethane, insbesondere 4-Isopropyldibenzoylmethan, 4-tert.-Butyl-4'-methoxydibenzoylmethan, Zimtsäureester, insbesondere p-Methoxyzimtsäure-2-ethylhexylester, p-Methoxyzimtsäureisoamylester, p-Methoxyzimtsäureoctylester, p-Methoxyzimtsäurepropylester, p-Aminobenzoesäure (PABA) und seinen Derivaten, insbesondere p-Aminobenzoesäureglycerolester, Butyl-PABA, Octyl-dimethyl-PABA, 2-Ethylhexylsalicylat, Homosalat, Octylsalicylat, Octyltriazon, Oxybenzon enthält.

3. Verwendung nach Anspruch 2, **dadurch gekennzeichnet, daß** eine Zusammensetzung eingesetzt wird, die als partikulären UV-Blocker ein oder mehrere anorganische Pigmente oder organische Pigmente enthält, das/die in der Lipidmatrix dispergiert und/oder an der Oberfläche der Lipidpartikel angelagert ist/sind.

4. Verwendung nach Anspruch 3, **dadurch gekennzeichnet, daß** das Pigment Bariumsulfat, Bentonite, Calciumcarbonat, Calciumsulfat, Eisen-(III)-oxide, Eisenoxidhydrat, Kaolin, Kohlenschwarz, Kupferoxid, Magnesiumoxid, Silber, Siliziumdioxid, insbesondere Aerosil, Syloid, hydrophobes alkyliertes Siliziumdioxid, Talcum, Titandioxid, Bismutoxychlorid, Zinkoxid, Zinkstearat oder Melanin einzeln oder in Mischung umfaßt.

5. Verwendung nach einem der Ansprüche 1 bis 4, **dadurch gekennzeichnet, daß** eine Zusammensetzung eingesetzt wird, die eine oder mehrere antioxidativ wirkende Substanzen einzeln oder in Mischung enthält, die in der Lipidmatrix gelöst und/oder dispergiert und/oder absorbiert und/oder an der Oberfläche der Lipidpartikel adsorbiert sind.

6. Verwendung nach Anspruch 5, **dadurch gekennzeichnet, daß** als antioxidativ wirkende Substanz Retinol, Retinolderivate, insbesondere Retinolpalmitat, Retinolacetat, Vitamin E, Vitamin E-Derivate, insbesondere Vitamin E-Acetat, Vitamin E-Linoleat, Vitamin E-Nicotinat, Vitamin E-Palmitat, Vitamin E-POE(22)succinat, Vitamin C, Vitamin C-Derivate, insbesondere Vitamin C-Palmitat, Magnesiumascorbat, Magnesiumphosphat, Aescin, Butylhydroxyanisol (BHA), Butylhydroxytoluol (BHT), Cystein, Dilaurylthiodipropionat, Dodecylgallat, Kaffeesäure, Liponsäure und Derivate, Propylgallat, Flavonoide, insbesondere Rutin oder ein Derivat desselben, Quercetin oder ein Derivat desselben, oder Gerbstoffe einzeln oder in Mischung verwendet wird/werden.

7. Verwendung nach einem der Ansprüche 1 bis 6, **dadurch gekennzeichnet, daß** eine Zusammensetzung eingesetzt wird, die zusätzlich hautpflegende Substanzen und/oder feuchtigkeitserhöhende Substanzen enthält, die in der Lipidmatrix gelöst und/oder dispergiert und/oder an der Oberfläche der Lipidpartikel adsorbiert sind.

8. Verwendung nach Anspruch 7, **dadurch gekennzeichnet, daß** als hautpflegende Substanz und/oder feuchtigkeitserhöhend Substanz Aminosäurederivate, insbesondere Arginin-Pyroglutaminat, Glutaminsäure, Lysin-Pyroglutaminat, Glucose, Glycerol, Harnstoff, Mucopolysaccharide, insbesondere Hyaluronsäure, Natriumlactat, Natrium-Pyrrolidoncarbonsäure, Propylenglycol, Vitamin A, insbesondere Retinöle oder ein Derivat derselben, Polysaccharide, Uronsäuren, Saccharoseglutamat, Allantoin, Biotin, Bisabolol, Cholesterol, Collagen oder ein Derivat desselben, Elastin, Glycoproteine, Hyaluronsäure oder ein Derivat derselben, Keratin oder ein Derivat desselben, Lecithin, Linolsäure, Linolensäure, Milchproteine, Niacinamid, Panthenol oder ein Derivat desselben, Riboflavin, Schwefel, Harnstoff, Sojabohnenöl, Tocopherol oder ein Derivat desselben, einzeln oder in Mischung eingesetzt wird/werden.

9. Verwendung nach einem der Ansprüche 1 bis 8, **dadurch gekennzeichnet, daß** eine Zusammensetzung eingesetzt wird, die zusätzlich natürliche, synthetische, halbsynthetische Duftstoffe einzeln oder in Mischung enthält, die in der Lipidmatrix gelöst und/oder dispergiert und/oder an der Oberfläche der Lipidpartikel adsorbiert sind.

10. Verwendung nach Anspruch 9, **dadurch gekennzeichnet, daß** die zusätzlichen natürlichen, synthetischen oder halbsynthetischen Duftstoffe ätherische Öle, Parfüms, Pheromone oder Repellents sind.

11. Verwendung nach Anspruch 10, **dadurch gekennzeichnet, daß** als ätherische Öle Zitronenöl, Rosenöl, Lavendelöl, Bergamottöl, Melissenöl, Nelkenöl, Zimtöl, Orangenöl, Jasminöl, Rosmarinöl, Anisöl, Pfefferminzöl, Sandelholzöl Ylang-Ylang-Öl oder deren isolierte Inhaltsstoffe, insbesondere 1,8-Cineol, Menthol, Terpinhydrat, Limonen, α-Pinen oder Eugenol eingesetzt wird.

12. Verwendung nach Anspruch 10 oder 11, **dadurch gekennzeichnet, daß** als Repellents natürliche Repellents, insbesondere Citrusöle, Eukalyptusöl und Campher, oder synthetische Repellents, insbesondere N,N-Diethyl-toluamid (DEET), Dibutylphthalat, Dimethylphthalat oder 2-Ethyl-1,3-hexandiol eingesetzt werden.

13. Verwendung nach einem der Ansprüche 1 bis 12, **dadurch gekennzeichnet, daß** als Lipid bei Raumtemperatur (20°C) feste Lipide wie natürliche und synthetische Mono-, Di- und Triglyceride, deren Mischungen, Fettalkohole, Ester und Ether derselben, einzeln oder in Mischung eingesetzt werden, insbesondere Cetylpalmitat, Glycerolmonostearat, Glycerolpalmitosteärat, Glycerolricinoleat, Glyceroltribehenat (Compritol), Glyceroltrilaurat, Hartfett (Witepsole), mikrokristalline Triglyceride (Dynasane), Stearylalkohol einzeln oder in Mischung enthalten.

14. Verwendung nach einem der Ansprüche 1 bis 13, **dadurch gekennzeichnet, daß** mindestens ein bei Raumtemperatur (20° C) festes Lipid eingesetzt wird, dem mindestens ein bei Raumtemperatur flüssiges Lipid zur Herstellung einer Lipidmischung (lipid blend) zugemischt ist.

15. Verwendung nach Anspruch 14, **dadurch gekennzeichnet, daß** als zugemischtes flüssiges Lipid (gesättigte, partial-gesättigte und ungesättigte) mittelkettige Triglyceride (MCT), insbesondere Miglyol, langkettige Trigylceride (LCT), insbesondere Isopropylmyristat, pflanzliche Öle, insbesondere Avocadoöl, Baumwollsamenöl, Distelöl, Erdnußöl, Jojobaöl, Kokosnußöl, Leinöl, Nußöl, Olivenöl, Palmkernöl, Sesamöl, Weizenkeimöl, tierische Öle, insbesondere wie Lebertran, Heilbuttleberöl, Rinderklauenöl, einzeln oder in Mischung eingesetzt werden.

16. Verwendung nach einem der Ansprüche 1 bis 15, **dadurch gekennzeichnet, daß** die Lipidpartikel durch Mahlen hergestellt wurden, insbesondere in einer Kugelmühle oder einer Mörsermühle oder durch Luftstrahlmahlung.

17. Verwendung nach einem der Ansprüche 1 bis 15, **dadurch gekennzeichnet, daß** die Lipidpartikel durch Dispergieren des Lipids in einer äußeren flüssigen Phase hergestellt wurden, wobei das Lipid dabei im festen und/oder flüssigen Zustand vorliegt.

18. Verwendung nach Anspruch 17 **dadurch gekennzeichnet, daß** das Lipid unterhalb seines Schmelzpunktes in einer äußeren Phase dispergiert worden ist, insbesondere durch eine Rotor-Stator-Kolloidmühle, einen hochtourigen Rührer, insbesondere eine Zahnscheibe, einen Hochdruckhomogenisator, insbesondere einen Kolben-Spalt Homogenisator oder mit einem Microfluidizer.

19. Verwendung nach Anspruch 17 **dadurch gekennzeichnet, daß** das Lipid in der Nähe oder oberhalb seines Schmelzpunktes in einer äußeren Phase dispergiert worden ist, insbesondere durch eine Rotor-Stator-Kolloidmühle, einen hochtourigen Rüher, insbesondere einen Ultra-Turrax, Silverson Rührer, eine Zahnscheibe, einen statischen Mischer im Mikromaßstab oder im Makromaßstab, einen Hochdruckhomogenisator, insbesondere einen Kolben-Spalt Homogenisator oder mit einem Microfluidizer.

20. Verwendung nach einem der Ansprüche 1 bis 19, **dadurch gekennzeichnet, daß** die in der äußeren flüssigen Phase dispergierten Lipidpartikel durch Tenside, Polymere oder Peptisatoren (Anti-Flokkulantien) stabilisiert sind und/oder durch Erhöhung der Viskosität der flüssigen Phase durch Zusatz von vikositätserhöhenden Stoffen eine Stabilisierung gegen Partikelaggregation bewirkt worden ist.

21. Verwendung nach Anspruch 20, **dadurch gekennzeichnet, daß** als Tenside Sorbitanfettsäureester wie Tween, Span, Zuckerester, insbesondere Saccharosestearat, Saccharosedistearat, Saccharoselaurat, Saccharoseoctanoat, Saccharosepalmitat, Saccharosemyristat, Fettalkohole, insbesondere Cetylstearyllalkohol, Natriumcetylstearylsulfat, Cocoamidopropylbetain, Natrium-Cocoamphoacetat, Polyglycerolmethylglucosedistearat, Lecithine, insbesondere Sojalecithin oder Eilecithin, Alkaliseifen, Metallseifen, insbesondere Calciumdilaurat, natürliche Tenside, insbesondere Saponine, einzeln oder in Mischung eingesetzt werden.

22. Verwendung nach Anspruch 20, **dadurch gekennzeichnet, daß** als Polymere Blockpolymere, insbesondere Poloxamer, Polyvinylderivate, insbesondere Polyvinylacetat, Polyvinylalkohol, Polyvinylpyrrolidon, Polystyrole, einzeln oder in Mischung eingesetzt werden.

23. Verwendung nach Anspruch 20, **dadurch gekennzeichnet, daß** als Peptisatoren (Anti-Flokkulantien), insbesondere Natriumcitrat, Natriumpyrophosphat oder Natriumsorbat, einzeln oder in Mischung eingestzt werden.

24. Verwendung nach Anspruch 20, **dadurch gekennzeichnet, daß** als viskositätserhöhende Substanzen Cellulosederivate, insbesondere Carboxymethylcellulose, Celluloseacetatphthalat, Hydroxyethylcellulose, Methylcellulose, Methylhydroxyethylcellulose, Methylhydroxypropylcellulose, Polyacrylate, Polyacrylsäuren, Polyvinylderivate, Alginate, Bentonit, hochdisperses Siliciumdioxid, Pektine, Tragant oder Xanthane einzeln oder in Mischung eingesetzt werden.

25. Verwendung nach einem der Ansprüche 1 bis 24 **dadurch gekennzeichnet, daß** in der äußeren Phase der Dispersion noch zusätzliche UV-blockierende Substanzen und/oder UV-blockierende Partikel, insbesondere Titandioxid, Zinkoxid, Melanin oder Silikate, insbesondere Aerosile, eingesetzt werden.

26. Verwendung nach einem der Ansprüche 1 bis 25 **dadurch gekennzeichnet, daß** das hergestellte Mittel in Form einer Formulierung zur Anwendung auf Haut und Schleimhaut vorliegt, insbesondere als Lotion, Creme, Salbe, Paste, Stift, insbesondere Lippenstift, oder Hautspray.

27. Verwendung nach einem der Ansprüche 1 bis 25 **dadurch gekennzeichnet, daß** das hergestellte Mittel in Form einer Formulierung zur Anwendung auf Haaren oder Kopfhaut vorliegt, insbesondere als Shampoo, Pflegekur oder wäßrige oder ölige Lotion.

## Claims

1. Use of solid, polymorphic crystalline or partially crystalline lipid particles or of a composition comprising solid, polymorphic crystalline or partially crystalline lipid particles, optionally in the form of a solid inner phase (lipid phase) dispersed in an outer liquid phase, as an agent for protection against UV-radiation, wherein the lipid particles have a size (average value of the main population) in the range of 10 nm to 1000 nm and exhibit an endothermic peak above 20 °C during the heating-up phase in heat calorimetry (DSC-differential scanning calorimetry), for the manufacture of agents with UV-radiation absorbing and/or reflecting action for application on the skin, mucous membranes, hair and scalp to protect against health-damaging UV radiation and for strengthening the natural skin barrier.

2. Use according to Claim 1, **characterised in that** a composition is added that further comprises one or a plurality of molecular and/or particulate UV blockers that are dissolved and/or dispersed in the lipid matrix material and/or adsorbed on the surface of the lipid particles, wherein it comprises one or a plurality of substances as the molecular UV-blocker, selected from benzophenone and its derivatives, 4-phenylbenzophenone, 2-hydroxy-4-n-octyloxybenzophenone, 2-hydroxy-4-methoxybenzophenone, 2,2'-dihydroxy-4,4'-dimethoxybenzophenone, sulisobenzone, benzimidazole derivatives, phenylbenzimidazole sulfonic acid, camphor derivatives, particularly 3-benzylidenecamphor, 3-(4-methylbenzylidenecamphor, terephthalylidenedicamphor sulfonic acid, dibenzoylmethanes, 4-isopropyldibenzoylmethane, 4-*tert*-butyl-4'-methoxydibenzoylmethane, cinnamic acid esters, 2-ethylhexyl ester of *p-*methoxycinnamic acid, isoamyl ester of *p*-methoxycinnamic acid, octyl ester of *p*-methoxycinnamic acid, propyl ester of *p*-methoxycinnamic acid, *p*-aminobenzoic acid (PABA) and its derivatives, glycerol esters of *p-*aminobenzoic acid, butyl-PABA, octyl-dimethyl-PABA, 2-ethylhexyl salicylate, homosalate, octyl salicylate, octyltriazone, and oxybenzone.

3. Use according to Claim 2, **characterised in that** a composition is added that comprises as the particulate UV blocker one or a plurality of inorganic pigments or organic pigments that is/are dispersed in the lipid matrix and/or is/are deposited on the surface of the lipid particles.

4. Use according to Claim 3, **characterised in that** the pigment comprises barium sulfate, bentonite, calcium carbonate, calcium sulphate, iron (III) oxides, iron oxide dihydrate, kaolin, carbon black, copper oxide, magnesium oxide, silver, silicon dioxide, particularly Aerosil, Syloid, hydrophobic alkylated silicon dioxide, talcum, titanium dioxide, bismuth oxychloride, zinc oxide, zinc stearate or melanin, individually or in a mixture.

5. Use according to one of Claims 1 to 4, **characterised in that** a composition is added that comprises one or a plurality of antioxidants individually or in a mixture, which are dissolved and/or dispersed and/or absorbed in the lipid matrix and/or adsorbed on the surface of the lipid particles.

6. Use according to Claim 5, **characterised in that** retinol, retinol derivatives, particularly retinol palmitate, retinol acetate, vitamin E, vitamin E derivatives, particularly vitamin E acetate, vitamin E linoleate, vitamin E nicotinate, vitamin E palmitate, vitamin E POE (22) succinate, vitamin C, vitamin C derivatives, particularly vitamin C palmitate, magnesium ascorbate, magnesium phosphate, aescine, butylhydroxyanisole (BHA), butylhydroxytoluene (BHT), cysteine, dilauryl thiodipropionate, dodecyl gallate, caffeic acid, liponic acid and derivatives, propyl gallate, flavonoids, particularly rutine or a derivative of rutine, quercetine, a derivative of quercetine, or tanning agents, individually or in a mixture is/are used as the antioxidant.

7. Use according to one of Claims 1 to 6, **characterised in that** a composition is added that further comprises a skin-care substance and/or moisturising substance that are dissolved and/or dispersed in the lipid matrix and/or are adsorbed on the surface of the lipid particles.

8. Use according to Claim 7, **characterised in that** amino acid derivatives, particularly arginine pyroglutaminate, glutamic acid, lysine pyroglutaminate, glucose, glycerol, urea, mucopolysaccharides, particularly hyaluronic acid, sodium lactate, sodium pyrrolidone carboxylic acid, propylene glycol, vitamin A, particularly retinols or a derivative of retinol, polysaccharides, uronic acids, saccharose glutamate, allantoin, biotin, bisabolol, cholesterol, collagen or a derivative of collagen, elastin, glycoproteins, hyaluronic acid or a derivative of hyaluronic acid, keratin or a derivative of keratin, lecithin, linoleic acid, linolenic acid, milk proteins, niacinamide, panthenol or a derivative of panthenol, riboflavin, sulfur, urea, soybean oil, tocopherol or a derivative of tocopherol are added as the skin-care substance and/or moisturising substance, individually or in a mixture.

9. Use according to one of Claims 1 to 8, **characterised in that** a composition is added that further comprises natural, synthetic or semi-synthetic fragrances, individually or in a mixture, which are dissolved and/or dispersed in the lipid matrix and/or adsorbed on the surface of the lipid particles.

10. Use according to Claim 9, **characterised in that** the additional natural, synthetic or semi-synthetic fragrances are ethereal oils, perfumes, pheromones or repellents.

11. Use according to claim 10, **characterised in that** lemon oil, rose oil, lavender oil, bergamot oil, balm mint oil, clove oil, cinnamon oil, orange oil, jasmine oil, rosemary oil, aniseed oil, peppermint oil, sandalwood oil, ylang-ylang oil or ingredients isolated from ylang-ylang oil, 1,8-cineole, menthol, terpinol hydrate, limonene, α-pinene or eugenol is added as the ethereal oil.

12. Use according to Claim 10 or 11, **characterised in that** natural repellents, particularly citrus oils, eucalyptus oil and camphor, or synthetic repellents, particularly N,N-diethyltoluamide (DEET), dibutyl phthalate, dimethyl phthalate or 2-ethyl-1,3-hexandiol are added as the repellents.

13. Use according to one of Claims 1 to 12, **characterised in that** solid lipids like natural and synthetic mono-, di- and triglycerides, their mixtures, fatty alcohols, esters and ethers thereof, are added singly or in mixtures as the room temperature (20 °C) solid lipids, in particular comprising cetyl palmitate, glycerol monostearate, glycerol palmitostearate, glycerol ricinoleate, glycerol tribehenate (Compritol), glycerol trilaurate, hard fat (Witepsole), microcrystalline triglycerides (Dynasane), stearyl alcohol singly or in mixtures.

14. Use according to one of Claims 1 to 13, **characterised in that** at least one room temperature (20 °C) solid lipid is added, to which at least one room temperature liquid lipid is mixed in, so as to produce a lipid mixture (lipid blend).

15. Use according to Claim 14, **characterised in that** (saturated, partially saturated and unsaturated) medium chain triglycerides (MCT), particularly Miglycol, long chain triglycerides (LCT), particularly isopropyl myristate, vegetal oils, particularly avocado oil, cotton-seed oil, thistle oil, peanut oil, jojoba oil, coconut oil, linseed oil, walnut oil, olive oil, palm-kernel oil, sesame oil, wheatgerm oil, animal oils, particularly cod-liver oil, halibut-liver oil, and neat's-foot oil, individually or in a mixture, are added as the mixed liquid lipid.

16. Use according to one of Claims 1 to 15, **characterised in that** the lipid particles were produced by grinding, particularly in a ball mill or a mortar grinder or by jet milling.

17. Use according to one of Claims 1 to 15, **characterised in that** the lipid particles were produced by dispersing the lipid in an external liquid phase, the lipid being in the solid and/or liquid state.

18. Use according to Claim 17, **characterised in that** the lipid has been dispersed below its melting point in an external phase, in particular by means of a rotor-stator colloid mill, a high-speed mixer, in particular a toothed disc mill, a high pressure homogeniser, in particular a high pressure plunger-valve homogeniser or a microfluidiser.

19. Use according to Claim 17, **characterised in that** the lipid has been dispersed at or close to or above its melting point in an external phase, in particular by means of a rotor-stator colloid mill, a high speed mixer, in particular an Ultra-Turrax, Silverson mixer, a toothed disc, a static mixer in a microscale or macroscale, a high pressure homogeniser, in particular a high pressure plunger-valve homogeniser or with a microfluidiser.

20. Use according to one of Claims 1 to 19, **characterised in that** the lipid particles, dispersed in the external liquid phase, are stabilised by surfactants, polymers or antiflocculants and/or a stabilisation against particle aggregation has been effected by increasing the viscosity of the liquid phase through the addition of viscosity-increasing substances.

21. Use according to Claim 20, **characterised in that** sorbitan fatty acid esters such as Tween, Span, sugar esters, particularly saccharose stearate, saccharose distearate, saccharose laurate, saccharose octanoate, saccharose palmitate, saccharose myristate, fatty alcohols; particularly cetylstearyl alcohol, sodium cetylstearyl sulphate, cocoamidopropylbetain, sodium cocoamphoacetate, polyglycerol methylglucose distearate, lecithins, particularly soybean lecithin or egg lecithin, alkaline soaps, metal soaps, particularly calcium dilaurate, natural surfactants, particularly saponins, individually or in a mixture are added as the surfactants.

22. Use according to Claim 20, **characterised in that** particularly polyoxamers, polyvinyl derivatives, particularly polyvinyl acetate, polyvinyl alcohol, polyvinyl pyrrolidone, and polystyrenes, individually or in a mixture, are added as the block polymers.

23. Use according to claim 20, **characterised in that** particularly sodium citrate, sodium pyrophosphate or sodium sorbate, individually or in a mixture, are added as the antiflocculant.

24. Use according to claim 20, **characterised in that** cellulose derivatives, particularly carboxymethyl cellulose, cellulose acetate phthalate, hydroxyethyl cellulose, methyl cellulose, methylhydroxyethyl cellulose, methylhydroxypropyl cellulose, polyacrylates, polyacrylic acids, polyvinyl derivatives, alginates, bentonite, highly-dispersed silicon dioxide, pectins, tragacanth or xanthan, individually or in a mixture, are added as the viscosity-increasing substance.

25. Use according to one of Claims 1 to 24, **characterised in that** further additional UV blocking substances and/or UV blocking particles, particularly titanium dioxide, zinc oxide, melanin or silicates, particularly aerosils, are added to the external phase of the dispersion.

26. Use according to one of Claims 1 to 25, **characterised in that** the manufactured agent is present in the form of a formulation for application on skin and mucous membranes, particularly as a lotion, cream, balm, paste, stick, particularly lipstick, or hair spray.

27. Use according to one of Claims 1 to 25, **characterised in that** the manufactured agent is present in the form of a formulation for application on hair or scalp, particular as shampoo, care lotion, aqueous or oily lotion.

## Revendications

1. Utilisation de particules lipidiques solides, polymorphes, cristallines ou partiellement cristallines ou d'une composition qui comprend des particules lipidiques solides, polymorphes, cristallines ou partiellement cristallines, éventuellement sous la forme d'une phase interne solide (phase lipidique) dispersée dans une phase externe liquide, comme agents de protection contre un rayonnement UV, les particules lipidiques présentant une taille, exprimée en valeur moyenne de la population dominante, comprise dans la plage de 10 nm à 1000 nm et montrant, pendant la phase de chauffage en calorimétrie (DSC - calorimétrie à balayage différentiel), un pic endothermique supérieur à 20°C, pour fabriquer des agents ayant un effet absorbant et/ou réfléchissant de rayonnement UV pour une application sur la peau, les muqueuses, les cheveux et le cuir chevelu, afin de les protéger contre un rayonnement nocif pour la santé et renforcer la barrière cutanée naturelle.

2. Utilisation selon la revendication 1, **caractérisée en ce que** l'on utilise une composition qui contient, en outre, un ou plusieurs agents moléculaires et/ou particulaires bloquant les UV, lesquels sont dissous et/ou dispersés dans le matériau de la matrice lipidique et/ou adsorbés à la surface des particules lipidiques, et à la composition contient comme agent bloquant les UV une ou plusieurs substances sélectionnées parmi la benzophénone et ses dérivés, en particulier, les composés 4-phénylbenzophénone, 2-hydroxy-4-n-octyloxybenzophénone, 2-hydroxy-4-méthoxybenzophénone, 2,2'-dihydroxy-4,4'-diméthoxybenzophénone, sulisobenzone, les dérivés de benzimidazole, en particulier, l'acide phénylbenzimidazolsulfonique, les dérivés du camphre, en particulier, le 3-benzylidène-camphre, le 3-(4-méthylbenzylidène)-camphre, l'acide téréphtalylidène-dicamphresulfonique, les dibenzoyl-méthanes, en particulier, le 4-isopropyldibenzoyl-méthane, le 4-tert-butyl-4'-méthoxydibenzoyl-méthane, les esters de l'acide cinnamique, en particulier, le 2-éthylhexylester de l'acide p-méthoxycinnamique, l'isoamylester de l'acide p-méthoxycinnamique, l'octylester de l'acide p-méthoxycinnamique, le propylester de l'acide p-méthoxycinnamique, l'acide p-aminobenzoïque (PABA) et ses dérivés, en particulier, l'ester glycérique de l'acide p-aminobenzoïque, le butyl-PABA, l'octyl-diméthyl-PABA, le salicylate de 2-éthylhexyle, l'homosalate, le salicylate d'octyle, l'octyltriazone et l'oxybenzone.

3. Utilisation selon la revendication 2, **caractérisée en ce que** l'on emploie une composition qui contient comme agents particulaires bloquant les UV un ou plusieurs pigments inorganiques ou organiques qui est/sont dispersé(s) dans la matrice lipidique et/ou qui est/sont fixé(s) sur la surface des particules lipidiques.

4. Utilisation selon la revendication 3, **caractérisée en ce que** le pigment comprend du sulfate de baryum, des bentonites, du carbonate de calcium, du sulfate de calcium, des oxydes de fer (III), de l'hydrate d'oxyde de fer, du kaolin, du noir de charbon, de l'oxyde de cuivre, de l'oxyde de magnésium, de l'argent, du dioxyde de silicium, en particulier, un Aerosil, du Syloid, du dioxyde de silicium hydrophobe alkylé, du talc, du dioxyde de titane, de l'oxychlorure de bismuth, de l'oxyde de zinc, du stéarate de zinc ou de la mélanine, individuellement ou en mélange.

5. Utilisation selon l'une quelconque des revendications 1 à 4, **caractérisée en ce que** l'on emploi une composition qui contient une ou plusieurs substances à effet anti-oxydant individuellement ou en mélange, qui sont dissoutes et/ou dispersées et/ou absorbées dans la matrice lipidique et/ou adsorbées à la surface des particules lipidiques.

6. Utilisation selon la revendication 5, **caractérisée en ce que** l'on utilise comme substance à effet anti-oxydant le rétinol, des dérivés du rétinol, en particulier, le palmitate de rétinol, l'acétate de rétinol, la vitamine E, des dérivés de la vitamine E, en particulier, l'acétate de vitamine E, le linoléate de vitamine E, le nicotinate de vitamine E, le palmitate de vitamine E, le succinate de vitamine E-POE(22), la vitamine C, des dérivés de vitamine C, en particulier, le palmitate de vitamine C, l'ascorbate de magnésium, le phosphate de magnésium, l'aescine, le butylhydroxyanisol (BHA), le butylhydroxytoluène (BHT), la cystéine, le thiodipropionate de dilauryle, le gallate de dodécyle, l'acide caféique, l'acide liponique et ses dérivés, le gallate de propyle, les flavonoïdes, en particulier, la rutine ou un de ses dérivés, la quercétine ou un de ses dérivés ou des tanins, individuellement ou en mélange.

7. Utilisation selon l'une quelconque des revendications 1 à 6, **caractérisée en ce que** l'on emploi une composition qui contient en outre des substances de soin de la peau et/ou des substances hydratantes, qui sont dissoutes et/ou dispersées dans la matrice lipidique et/ou adsorbées à la surface des particules lipidiques.

8. Utilisation selon la revendication 7, **caractérisée en ce que** l'on emploi comme substance de soin de la peau et/ou comme substance hydratante des dérivés d'acide aminé, en particulier, le pyroglutamate d'arginine, l'acide glutamique, le pyroglutamate de lysine, le glucose, le glycérol, l'urée, des mucopolysaccharides, en particulier, l'acide hyaluronique, le lactate de sodium, l'acide pyrrolidonecarboxylique de sodium, le propylèneglycol, la vitamine A, en particulier, des rétinols ou un de leurs dérivés, des polysaccharides, des acides uroniques, le glutamate de saccharose, l'allantoïne, la biotine, le bisabolol, le cholestérol, le collagène ou un de ses dérivés, l'élastine, des glycoprotéines, l'acide hyaluronique ou un de ses dérivés, la kératine ou un de ses dérivés, la lécithine, l'acide linoléique, l'acide linolénique, des protéines du lait, le niacinamide, le panthénol ou un de ses dérivés, la riboflavine, le soufre, l'urée, l'huile de graine de soja, le tocophérol ou un de ses dérivés, individuellement ou en mélange.

9. Utilisation selon l'une quelconque des revendications 1 à 8, **caractérisée en ce que** l'on emploi une composition qui contient en outre des matières odoriférantes naturelles, synthétiques, semi-synthétiques, individuellement ou en mélange, qui sont dissoutes et/ou dispersées dans la matrice lipidique et/ou adsorbées sur la surface des particules lipidiques.

10. Utilisation selon la revendication 9, **caractérisée en ce que** les matières odoriférantes naturelles, synthétiques ou semi-synthétiques supplémentaires sont des huiles essentielles, des parfums, des phéromones ou des répulsifs.

11. Utilisation selon la revendication 10, **caractérisée en ce que** l'on emploi comme huiles essentielles, de l'essence de citron, de l'essence de rose, de l'essence de lavande, de l'essence de bergamote, de l'essence de mélisse, de l'essence d'oeillet, de l'essence de cannelle, de l'essence d'orange, de l'essence de jasmin, de l'essence de romarin, de l'essence d'anis, de l'essence de menthe poivrée, de l'essence de bois de santal, de l'essence d'ylang-ylang ou leurs ingrédients isolés, en particulier, le 1,8-cinéol, le menthol, l'hydrate de terpine, le limonène, l'alpha-pinène ou l'eugénol.

12. Utilisation selon les revendications 10 ou 11,
**caractérisée en ce que** l'on emploi comme répulsifs des répulsifs naturels, en particulier, des huiles de citrus, de l'essence d'eucalyptus et du camphre ou des répulsifs synthétiques, en particulier, du N,N-diéthyltoluamide (DEET), du phtalate de dibutyle, du phtalate de diméthyle ou du 2-éthyl-1,3-hexanediol.

13. Utilisation selon l'une quelconque des revendications 1 à 12, **caractérisée en ce que** l'on emploi comme lipide des lipides solides à température ambiante (20°C), tels que des mono-, di- et triglycérides naturels et synthétiques, leurs mélanges, des alcools gras, leurs esters et leurs éthers, seuls ou en mélange, en particulier ceux comprenant, du palmitate de cétyle, du monostéarate de glycérol, du palmitostéarate de glycérol, du ricinoléate de glycérol, du tribéhénate de glycérol (Compritol), du trilaurate de glycérol, du gras dur (des Witepsols), des triglycérides microcristallins (des Dynasans), de l'alcool de stéaryle, individuellement ou en mélange.

14. Utilisation selon l'une quelconque des revendications 1 à 13, **caractérisée en ce que** l'on emploie au moins un lipide solide à température ambiante (20°C), auquel on ajoute en mélange au moins un lipide liquide à température ambiante pour fabriquer un mélange de lipides (lipid blend).

15. Utilisation selon la revendication 14, **caractérisée en ce que** l'on utilise comme lipide liquide ajouté en mélange des triglycérides à chaîne moyenne (saturés, partiellement saturés et insaturés) (MCT), en particulier du Miglyol, des triglycérides à chaîne longue (LCT), en particulier du myristate d'isopropyle, des huiles végétales, en particulier de l'huile d'avocat, de l'huile de graine de coton, de l'huile de chardon, de l'huile d'arachide, de l'huile de jojoba, de l'huile de noix de coco, de l'huile de lin, de l'huile de noix, de l'huile d'olive, de l'huile de palme, de l'huile de sésame, de l'huile de germe de blé, des huiles animales, en particulier de l'huile de foie de morue, de l'huile de flétan, de l'huile de pied de boeuf, individuellement ou en mélange.

16. Utilisation selon l'une quelconque des revendications 1 à 15, **caractérisée en ce que** les particules lipidiques ont été fabriquées par broyage, en particulier, dans un broyeur à boulets ou un broyeur à mortier ou par broyage à jet d'air.

17. Utilisation selon l'une quelconque des revendications 1 à 15, **caractérisée en ce que** les particules lipidiques ont été fabriquées en dispersant le lipide dans une phase liquide externe, où le lipide se présente, en l'occurrence, à l'état solide et/ou liquide.

18. Utilisation selon la revendication 17, **caractérisée en ce que** le lipide a été dispersé en dessous de son point de fusion dans une phase externe, en particulier, au moyen d'un moulin à colloïdes de type rotor-stator, d'un agitateur à grande vitesse de rotation, en particulier, un disque denté, d'un homogénéisateur à haute pression, en particulier, un homogénéisateur à écartement de piston ou d'un microfluidiseur.

19. Utilisation selon la revendication 17, **caractérisée en ce que** le lipide a été dispersé à peu près à son point de fusion ou au-dessus, dans une phase externe, en particulier, au moyen d'un broyeur à colloïdes de type rotor-stator, d'un agitateur à grande vitesse de rotation, en particulier un appareil de type Ultra-Turrax, un agitateur Silverson, un disque denté, d'un mélangeur statique à échelle microscopique ou à échelle macroscopique, d'un homogénéisateur à haute pression, en particulier, un homogénéisateur à écartement de piston ou d'un microfluidiseur.

20. Utilisation selon l'une quelconque des revendications 1 à 19, **caractérisée en ce que** les particules lipidiques dispersées dans la phase externe liquide sont stabilisées par des tensioactifs, des polymères ou des agents peptisants (agents anti-floculants) et/ou en provoquant, par l'augmentation de la viscosité de la phase liquide en ajoutant des substances augmentant la viscosité, une stabilisation contre de l'agrégation des particules.

21. Utilisation selon la revendication 20, **caractérisée en ce que** l'on emploi comme tensioactifs des esters d'acides gras de sorbitane, tels que le Tween, le Span, des esters de sucre, en particulier, le stéarate de saccharose, le distéarate de saccharose, le laurate de saccharose, l'octanoate de saccharose, le palmitate de saccharose, le myristate de saccharose, des alcools gras, en particulier, l'alcool de cétylstéaryle, le cétylstéarylesulfate de sodium, la cocoamidopropylbétaïne, le cocoamphoacétate de sodium, le distéarate de polyglycérolméthylglucose, des lécithines, en particulier, la lécithine de soja ou la lécithine d'oeuf, des savons de métaux alcalins, des savons de métaux, en particulier, le dilaurate de calcium, des tensioactifs naturels, en particulier, des saponines, individuellement ou en mélange.

22. Utilisation selon la revendication 20, **caractérisée en ce que** l'on emploie comme polymères des polymères séquencés, en particulier, le poloxamère, des dérivés de polyvinyle, en particulier, du poly(acétate de vinyle), de l'alcool polyvinylique, de la polyvinylpyrrolidone, des polystyrènes, individuellement ou en mélange.

23. Utilisation selon la revendication 20, **caractérisée en ce que** l'on emploi comme agents peptisants (agents anti-floculants), en particulier, du citrate de sodium, du pyrophosphate de sodium ou du sorbate de sodium, individuellement ou en mélange.

24. Utilisation selon la revendication 20, **caractérisée en ce que** l'on emploi comme substances augmentant la viscosité des dérivés de cellulose, en particulier, de la carboxyméthylcellulose, du phtalate acétate de cellulose, de l'hydroxyéthylcellulose, de la méthylcellulose, de la méthylhydroxyéthylcellulose, de la méthylhydroxypropylcellulose, des polyacrylates, des acides polyacryliques, des dérivés de polyvinyle, des alginates, de la bentonite, du dioxyde de silicium hautement dispersé, des pectines, de la gomme tragacante ou du xanthane, individuellement ou en mélange.

25. Utilisation selon l'une quelconque des revendications 1 à 24, **caractérisée en ce que** l'on emploie encore dans la phase externe de la dispersion des substances supplémentaires bloquant les UV et/ou des particules bloquant les UV, en particulier, du dioxyde de titane, de l'oxyde de zinc, de la mélanine ou des silicates, en particulier, des Aerosils.

26. Utilisation selon l'une quelconque des revendications 1 à 25, **caractérisée en ce que** le produit fabriqué se présente sous la forme d'une formulation à appliquer sur la peau et les muqueuses, en particulier, sous la forme d'une lotion, d'une crème, d'une pommade, d'une pâte, d'un bâton, en particulier, d'un bâton pour lèvres ou sous forme d'un pulvérisateur pour la peau.

27. Utilisation selon l'une quelconque des revendications 1 à 25, **caractérisée en ce que** le produit fabriqué se présente sous la forme d'une formulation à appliquer sur les cheveux ou le cuir chevelu, en particulier, comme shampooing, soin de cure ou lotion aqueuse ou huileuse.
